# FASCICULE DE BREVET EUROPEEN

(11) **EP 1 228 091 B1**
(45) Date de publication et mention de la délivrance du brevet: **28.04.2010**
(21) Numéro de dépôt: 00974648.8
(22) Date de dépôt: 03.11.2000
(51) Int. Cl.: C07K 14/47, C12N 15/63, C12Q 1/68

(54) **PROTEINES MEMBRANAIRES CTL (CHOLINE TRANSPORTER LIKE) IMPLIQUEES DANS LE TRANSPORT DE LA CHOLINE**
MEMBRANE-PROTEINE CTL (CHOLINE TRANSPORTER LIKE) DIE IN DEM TRANSPORT DER CHOLINE INVOLVIERT SIND
CHOLINE TRANSPORTER LIKE (CTL) MEMBRANE PROTEINS INVOLVED IN CHOLINE TRANSPORT

(30) Priorité: 05.11.1999 FR 9913883
(43) Date de publication de la demande: 07.08.2002
(73) Titulaire: CENTRE NATIONAL DE LA RECHERCHE SCIENTIFIQUE (CNRS), 75794 Paris Cedex 16 (FR)
(72) Inventeur: O'REGAN, Seana, F-78350 Les Loges en Josas (FR); MEUNIER, François, F-91190 Gif sur Yvette (FR); TRAIFFORT, Elisabeth, F-75014 Paris (FR); RUAT, Martial, F-92340 Bourg la Reine (FR)
(74) Mandataire: Ahner, Francis
(86) Numéro de dépôt international: PCT/FR2000/003069
(87) Numéro de publication internationale: WO 2001/032704

(56) Documents cités:
- WO-A-98/07859
- WO-A-98/27205
- WO-A-98/45435
- WO-A-98/45436
- WO-A-99/06439
- WO-A-99/26973
- NIKAWA J-I ET AL: "CLONING OF A GENE ENCODING CHOLINE TRANSPORT IN SACCHAROMYCES-CEREVISIAE" JOURNAL OF BACTERIOLOGY, vol. 166, no. 1, 1986, pages 328-330, XP000925069 ISSN: 0021-9193 cité dans la demande
- MAYSER W ET AL: "PRIMARY STRUCTURE AND FUNCTIONAL EXPRESSION OF A CHOLINE TRANSPORTER EXPRESSED IN THE RAT NERVOUS SYSTEM" FEBS (FEDERATION OF EUROPEAN BIOCHEMICAL SOCIETIES) LETTERS, vol. 305, no. 1, 1992, pages 31-36, XP000915418 ISSN: 0014-5793
- VAROQUI HELENE ET AL: "Cloning and expression of the vesamicol binding protein from the marine ray Torpedo: Homology with the putative vesicular acetylcholine transporter (UNC-17) from Caenorhabditis elegans." FEBS (FEDERATION OF EUROPEAN BIOCHEMICAL SOCIETIES) LETTERS, vol. 342, no. 1, 1994, pages 97-102, XP000915417 ISSN: 0014-5793
- DATABASE EMBL NUCLEOTIDE AND PROTEIN SEQUENCES, 30 juin 1999 (1999-06-30), XP002142598 HINXTON, GB
- DATABASE EMBL NUCLEOTIDE AND PROTEIN SEQUENCES, 18 août 1998 (1998-08-18), XP002142599 HINXTON, GB
- DATABASE EMBL NUCLEOTIDE AND PROTEIN SEQUENCES, 6 février 1998 (1998-02-06), XP002142600 HINXTON, GB
- DATABASE EMBL NUCLEOTIDE AND PROTEIN SEQUENCES, 18 février 1996 (1996-02-18), XP002142601 HINXTON, GB
- DATABASE EMBL NUCLEOTIDE AND PROTEIN SEQUENCES, 1 novembre 1999 (1999-11-01), XP002142602 HINXTON, GB
- O'REGAN SEANA ET AL: "An electric lobe suppressor for a yeast choline transport mutation belongs to a new family of transporter-like proteins." PROCEEDINGS OF THE NATIONAL ACADEMY OF SCIENCES OF THE UNITED STATES, vol. 97, no. 4, 15 février 2000 (2000-02-15), pages 1835-1840, XP002142603 Feb. 15, 2000 ISSN: 0027-8424

## Description

La présente invention concerne l'identification d'une nouvelle famille de gènes CTL (Choline Transporter Like), en particulier de hCTL1 et hCTL2, impliquée dans le métabolisme et/ou le transport de choline dans les cellules telles que les cellules du tractus intestinal, les cellules nerveuses, notamment les motoneurones, les neurones sensitifs, les neurones du noyau dorsal de la moelle épinière et les oligodendrocytes. L'invention ouvre de nouvelles perspectives notamment pour le traitement de la dysautonomie familiale, et de la maladie de Tangier. Plus généralement, l'identification des gènes CTL permet la mise au point de nouvelles stratégies pour le traitement des maladies du système nerveux, notamment des maladies neurodégénératives, démyélinisantes, de préférence la maladie d'Alzheimer, de Parkinson, et de Huntington.

La choline est un métabolite qui contribue à la production des membranes via la phosphatidylcholine. Ce métabolite joue également un rôle important dans les neurones cholinergiques, où il participe à la synthèse du neurotransmetteur acétylcholine. Dans certaines cellules, la phosphatidylcholine peut être produite par méthylation de la phosphatidyléthanolamine, et dans les organismes unicellulaires, les plantes et les animaux, la choline libre est absorbée comme nutriment. L'absorption de choline, dépendante du sodium et couplée à la synthèse de l'acétylcholine dans les terminaisons des nerfs cholinergiques, est particulièrement bien caractérisée au niveau fonctionnel (1-3), mais a échappé jusqu'à présent à divers essais d'identification basés sur la purification de protéines (4) même conjointement à l'utilisation d'un ligand sélectif et irréversible.

Dans le cadre de la présente invention, on a cloné un gène suppresseur de la mutation du transport de la choline chez la levure provenant d'une librairie d'expression réalisée à partir de l'ADNc provenant du lobe électrique de torpille. On a ensuite isolé le gène homologue chez le rat, rCTL1, qui se trouve fortement exprimé sous la forme d'un produit de transcription de 3,5 kb dans la moelle épinière et le cerveau, et sous la forme d'un ARNm de 5 kb dans le colon. Une hybridation in situ a montré une forte expression de rCTL1 dans les neurones moteurs, sensitifs et du noyau dorsal de la moelle épinière du rat et les oligodendrocytes, et dans une moindre mesure dans diverses populations neuronales réparties dans tout le cerveau. Dans les tissus périphériques, on a identifié des niveaux élevés de rCTL1 dans la couche cellulaire de la muqueuse du colon. Chez l'homme, le gène hCTL1 est associé à des marqueurs localisés en 9q.31.2, proches des locus de la dysautonomie familiale et de la maladie de Tangier. On a également trouvé plusieurs gènes homologues chez les mammifères (CTL2-4). La localisation de hCTL2 en 19p13.1 et de hCTL4 en 6p21.3 indique que les protéines CTL s'ajoutent à un certain nombre d'autres familles de gènes connues pour avoir été dupliquées à ces loci. Tous les gènes homologues de CTL1 codent pour des protéines comprenant 10 domaines transmembranaires putatifs, dont 2 contiennent des motifs de type transporteur.

Ainsi, l'identification et la caractérisation de cette famille de protéines, ci-après désignée CTL, ouvre de nouvelles perspectives notamment pour le traitement de la dysautonomie familiale, maladie qui inclut un composant cholinergique périphérique (27) avec des manifestations tant autonomes que motrices à la naissance, et une démyélinisation progressive du SNC chez les adultes (28) ; et de la maladie de Tangier (30) et (31). Plus généralement, l'identification des gènes CTL permet d'envisager de nouvelles thérapies pour l'ensemble des maladies impliquant les cellules exprimant les transcrits d'un gène CTL telles que les cellules du tractus intestinal, les cellules nerveuses, notamment les motoneurones; les neurones sensitifs, les neurones du noyau dorsal de la moelle épinière et les oligodendrocytes, notamment des maladies neurodégénératives, démyélinisantes, de la maladie d'Alzheimer, de Parkinson, et de Huntington.

### Description

Ainsi, la présente invention concerne un acide nucléique purifié ou isolé, **caractérisé en ce qu**'il comprend une séquence nucléique choisie parmi le groupe de séquences suivantes :
a) la séquence SEQ ID No. 1 (hCTL1) ;
b) la séquence SEQ ID No. 2 (hCTL2) ;
c) une séquence nucléique présentant un pourcentage d'identité globale d'au moins 80 %, 90%, 95% ou 99% après alignement optimal avec une séquence telle que définie en a), b) ou c) ;
d) la séquence complémentaire ou la séquence de l'ARN correspondant à une séquence telle que définie en a), b), c) ou d).

La SEQ ID N°1 est la séquence codante pour hCTL1 telle que représentée ci-dessous:

On entendra désigner par « hCTL1 » le gène codant pour les deux formes polypeptidiques issues de l'épissage alternatif CTL1a et CTL1b.

La SEQ ID N°2 est la séquence codante pour hCTL2 telle que représentée ci-dessous :

Par acide nucléique, séquence nucléique ou d'acide nucléique, polynucléotide, oligonucléotide, séquence de polynucléotide, séquence nucléotidique, termes qui seront employés indifféremment dans la présente description, on entendra désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin, ainsi que des produits de transcription desdits ADNs. Un nucléotide naturel d'acide nucléique se définit en ce que la base azotée est choisie parmi l'adénine, la guanine, l'uracile, la cytosine, et la thymine. Un nucléotide peut être modifié au niveau des bases. On peut citer notamment l'inosine, la méthyl-5- désoxycytidine, désoxyuridine, la dimétylamino-5- désoxyuridine, la diamino-2,6- purine, la bromo-5-désoxyuridine ou toute autre base modifiée capable d'hybridation.

Il doit être compris que la présente invention ne concerne pas les séquences nucléotidiques dans leur environnement chromosomique naturel, c'est-à-dire à l'état naturel. Il s'agit de séquences qui ont été isolées et/ou purifiées, c'est-à-dire qu'elles ont été prélevées directement ou indirectement, par exemple par copie, leur environnement ayant été au moins partiellement modifié.

Par " pourcentage d'identité globale" entre deux séquences d'acide nucléique ou d'acides aminés au sens de la présente invention, on entend désigner un pourcentage de nucléotides ou de résidus d'acides aminés identiques entre les deux séquences complètes à comparer, obtenu après le meilleur alignement, ce pourcentage étant purement statistique et les différences entre les deux séquences étant réparties au hasard et sur toute leur longueur. Les comparaisons de séquences entre deux séquences d'acide nucléique ou d'acides aminés sont traditionnellement réalisées en comparant ces séquences complètes après les avoir alignées de manière optimale, ladite comparaison étant réalisée par segment ou par " fenêtre de comparaison " pour identifier et comparer les régions locales de similarité de séquence. L'alignement optimal des séquences pour la comparaison peut être réalisé, outre manuellement, au moyen de l'algorithme d'homologie locale de Smith et Waterman (1981) [Ad. App. Math. 2 : 482], au moyen de l'algorithme d'homologie locale de Neddleman et Wunsch (1970) [J. Mol. Biol. 48 : 443], au moyen de la méthode de recherche de similarité de Pearson et Lipman (1988) [Proc. Natl. Acad. Sci. USA 85 : 2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI) et (DNASIS,Version 2.5 pour Windows ; Hitachi Software Engineering Co., Ltd, South San Francisco, CA, en utilisant les paramètres standards décrits dans le manuel du fabriquant).

Dans ce contexte, les séquences et les pourcentages d'identité peuvent également être obtenus en utilisant les ressources informatiques internet. On peut citer les programmes Blast (WWW.ncbi.nlm.nih.gov) et le programme FastDB avec les paramètres suivants " Mismatch penalty 1.00 ; Gap Penalty 1.00 ; Gap Size Penalty 0.33 ; joining penalty 30.0. Ces algorithmes sont présentés dans Current Methods in Sequencing and synthesis Mehtods and Applications, pages 127-149, 1988, Ala R. Liss, Inc.

Le pourcentage d'identité entre deux séquences d'acide nucléique ou d'acides aminés est déterminé en comparant ces deux séquences alignées de manière optimale par " fenêtre de comparaison " dans laquelle la région de la séquence d'acide nucléique ou d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par séquences nucléiques présentant un pourcentage d'identité d'au moins 80 %, 90 %, 95 % ou 99 %, après alignement optimal avec une séquence de référence, on entendra désigner les séquences nucléiques présentant, par rapport à la séquence nucléique de référence certaines modifications comme en particulier une délétion, une troncation, un allongement, une fusion chimérique, et/ou une substitution, notamment ponctuelle, et dont la séquence nucléique présente au moins 80 %, 90 %, 95 % ou 99 %, d'identité après alignement optimal avec la séquence nucléique de référence. Il s'agit de préférence de séquences dont les séquences complémentaires sont susceptibles de s'hybrider spécifiquement avec la séquence SEQ ID No. 1 ou 2 de l'invention. De préférence, les conditions d'hybridation spécifiques ou de forte stringence seront telles qu'elles assurent au moins 80 %, 90 %, 95 % ou 99 % d'identité après alignement entre l'une des deux séquences et la séquence complémentaire de l'autre.

Une hybridation dans des conditions de forte stringence signifie que les conditions de température et de force ionique sont choisies de telle manière qu'elles permettent le maintien de l'hybridation entre deux fragments d'ADN complémentaires. A. titre illustratif, des conditions de forte stringence de l'étape d'hybridation aux fins de définir les fragments polynucléotidiques décrits ci-dessus, sont avantageusement les suivantes. L'hybridation ADN-ADN ou ADN-ARN est réalisée en deux étapes : (1) préhybridation à 42°C pendant 3 heures en tampon phosphate (20 mM, pH 7,5) contenant 5 x SSC (1 x SSC correspond à une solution 0,15 M NaCl + 0,015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille > 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-dessus pour un polynucléotide de taille définie, seront adaptées par l'homme du métier pour des oligonucléotides de taille plus grande ou plus petite, selon l'enseignement de Sambrook et al. Molecular Cloning A Laboratory Manual (Cold Spring Harbor Press, 1989) aux paragraphes 11.1 à 11.61.

Parmi les séquences nucléiques présentant un pourcentage d'identité globale d'au moins 80 %, 90 %, 95 % ou 99 %, après alignement optimal avec la séquence selon l'invention, on préfère également les séquences nucléiques variantes de la séquence nucléique SEQ ID No. 1 ou 2, ou de ses fragments, c'est-à-dire l'ensemble des séquences nucléiques correspondant à des variants alléliques, c'est-à-dire des variations individuelles de la séquence nucléique SEQ ID No. 1 ou 2. Ces séquences mutées naturelles correspondent à des polymorphismes présents chez les mammifères, en particulier chez l'être humain et, notamment, à des polymorphismes pouvant conduire à la survenue d'une pathologie. De préférence, la présente invention concerne les séquences nucléiques variantes dans lesquelles les mutations conduisent à une modification de la séquence d'acides aminés du polypeptide, ou de ses fragments, codé par la séquence normale de séquence SEQ ID No. 1 ou 2.

De préférence, l'acide nucléique selon l'invention est constitué de la séquence SEQ ID No. 1, la séquence complémentaire ou de la séquence de l'ARN correspondant de la séquence SEQ ID No. 1 ou de la séquence SEQ ID No. 2, la séquence complémentaire ou de la séquence de l'ARN correspondant de la séquence SEQ ID No. 2.
Ledit acide nucléique peut comporter toute séquence codante pour un polypeptide sélectionné parmi hCTL1b de séquence SEQ ID N°3, hCTL1a de séquence SEQ ID N°9 et hCTL2 de séquence SEQ ID N°4.

Un second aspect de l'invention porte sur un polypeptide **caractérisé en ce qu**'il comprend une séquence peptidique ayant au moins 80%, de préférence 90%, 95% ou 99% d'identité après alignement optimal avec une séquence sélectionnée parmi les SEQ ID N°3, 4 et 9, ledit polypeptide étant impliqué dans le métabolisme et/ou le transport de choline dans les cellules, en particulier dans les cellules nerveuses. De préférence, ledit polypeptide possède une séquence sélectionnée parmi les SEQ ID N°3, 4, et 9.

La séquence SEQ ID N°3 suivante représente le polypeptide hCTL1b:

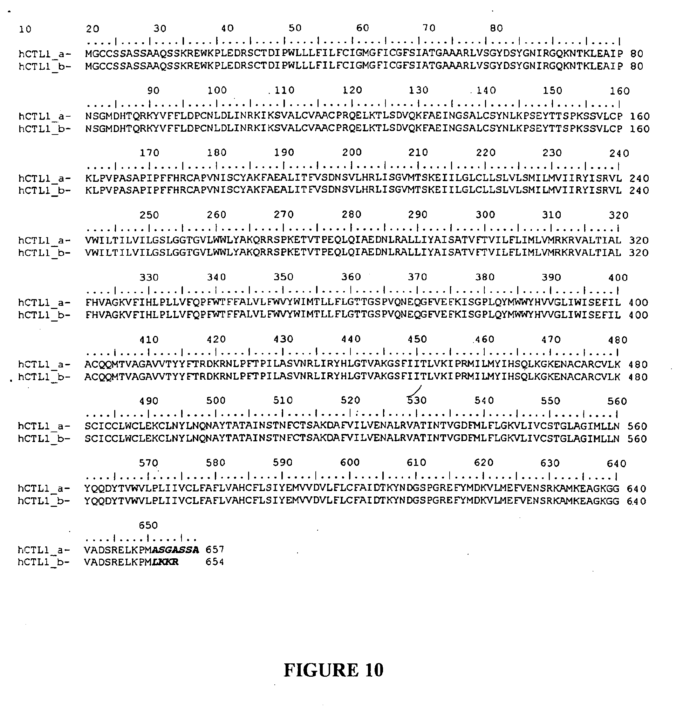

La SEQ ID N°9 (hCTL1a) correspond à une autre forme issues d'un épissage alternatif du gène hCTL1. Elle diffère de hCTL1b seulement en son extrémité C-terminale. Les séquences précédant le codon stop sont respectivement MLKKR pour hCTL1b et MASGASSA pour hCTL1a (Figure 10). Sauf mention particulière, on entendra désigner par protéine ou polypeptide hCTL1 aussi bien hCTL1a que hCTL1b.

La séquence SEQ ID N°4 suivante représente le polypeptide hCTL2 :

Ces polypeptides se **caractérisent en ce que** leur substrat est la choline. En ce sens, ils sont impliqués dans la production de l'acétylcholine et/ou dans la production des composants phospholipidiques de la membrane des cellules, notamment des cellules du tractus intestinales, des cellules nerveuses telles que les motoneurones, les neurones sensitifs, les neurones du noyau dorsale de la moelle épinière et les oligodendrocytes.

Un autre aspect de l'invention a trait à un vecteur comprenant une séquence nucléotidique telle que définie ci-dessus. Dans ce vecteur, ladite séquence peut être fusionnée à un promoteur efficace dans les cellules eucaryotes et/ou procaryotes et/ou peut comporter en outre un gène de sélection. Ainsi, une cellule transformée par ledit vecteur est également visée. Parmi les cellules que l'on peut transformées, on peut citer bien entendu les cellules bactériennes (Olins et Lee, 1993), mais également les cellules de levure (Buckholz, 1993), de même que les cellules animales, en particulier les cultures de cellules de mammifères (Edwards et Aruffo, 1993), et notamment les cellules d'ovaire de hamster chinois (CHO), mais également les cellules d'insectes dans lesquelles on peut utiliser des procédés mettant en oeuvre des baculovirus par exemple (Luckow, 1993).

L'invention se rapporte également à un anticorps capable de se lier spécifiquement à un polypeptide mentionné ci-dessus, ayant une séquence sélectionnée parmi les SEQ ID N°3, 4 et 9.

Un aspect supplémentaire de l'invention concerne un procédé permettant l'amplification et/ou la détection d'un acide nucléique définie ci-dessus dans un échantillon **caractérisé en ce qu**'on utilise comme amorce et/ou sonde au moins un oligonucléotide comprenant au moins 12 nucléotides consécutifs, de préférence 15, 20, 30 ou 50 nucléotides consécutifs d'une séquence choisie parmi les séquences suivantes :
a) la séquence SEQ ID N°2 ;
b) une séquence nucléique présentant un pourcentage d'identité globale d'au moins 80 % sur toute sa longueur après alignement optimal avec une séquence telle que définie en a), ladite séquence codant un polypeptide impliqué dans le métabolisme et/ou le transport de choline dans les cellules, en particulier dans les cellules nerveuses ;
c) la séquence complémentaire ou la séquence de l'ARN correspondant à une séquence telle que définie en a) ou b).
ou d'une séquence capable de s'y hybrider. Avantageusement, une telle séquence est capable de reconnaître spécifiquement hCTL1a, hCTL1b et hCTL2.

On entend par « mutations » des délétions, des insertions, des mutations ponctuelles qui peuvent aussi bien être dans les régions codantes et non codantes des gènes CTL (intron, promoteur, enhancer ou silencer). Ainsi, il est possible d'identifier des mutations affectant l'activité de la protéine CTL, l'épissage du gène et son niveau d'expression.

Une " sonde " se définie, dans le sens de l'invention, comme étant un fragment nucléotidique comprenant par exemple de 12 à 100 nucléotides, notamment de 15 à 35 nucléotides, possédant une spécificité d'hybridation dans des conditions déterminées pour former un complexe d'hybridation avec un acide nucléique cible. Les sondes selon l'invention, qu'elle soient spécifiques ou non-spécifiques, peuvent être immobilisées, directement ou indirectement, sur un support solide ; on parle alors de " sonde de capture ". Par ailleurs, lesdites sondes peuvent porter un agent marqueur permettant leur détection ; on parle alors de " sonde de détection ".

Une " sonde de capture " est immobilisée ou immobilisable sur un support solide par tout moyen approprié, par exemple par covalence, par adsorption, ou par synthèse directe sur un support solide. Ces techniques sont notamment décrites dans la demande de brevet WO 92/10092. La méthode la plus générale consiste à immobiliser l'acide nucléique extrait des cellules de différents tissus ou de cellules en culture sur un support (tels que la nitrocellulose, le nylon, le polystyrène) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde). On peut citer également comme support solide, les puces à ADN, notamment les puces commercialisées par Affymetrix, Cis Bio International / LETI.

Une " sonde de détection " peut être marquée au moyen d'un marqueur choisi par exemple parmi les isotopes radioactifs, des enzymes, en particulier des enzymes susceptibles d'agir sur un substrat chromogène, fluorigène ou luminescent (notamment une peroxydase ou une phosphatase alcaline), des composés chimiques chromophores, des composés chromogènes, fluorigènes ou luminescents, des analogues de base nucléotitiques, et des ligands tels que la biotine. Le marquage des amorces ou des sondes selon l'invention est réalisé par des éléments radioactifs ou par des molécules non radioactives. Parmi les isotopes radioactifs utilisés, on peut citer le 32P, le 33P, le 35S, le 3H ou le 1251. Les entités non radioactives sont sélectionnées parmi les ligands tels la biotine, l'avidine, la streptavidine, la dioxygénine, les haptènes, les colorants, les agents luminescents tels que les agents radioluminescents, chémiluminescents, bioluminescents, fluorescents, phosphorescents.

Les polynucléotides selon l'invention peuvent ainsi être utilisés comme amorce et/ou sonde dans des procédés mettant en oeuvre notamment la technique de PCR (réaction en chaîne à la polymérase) (Erlich, 1989 ; Innis et al., 1990, et Rolfs et al., 1991). Cette technique nécessite le choix de paires d'amorces oligonucléotidiques encadrant le fragment qui doit être amplifié. On peut, par exemple, se référer à la technique décrite dans le brevet américain U.S. N° 4,683,202. Les fragments amplifiés peuvent être identifiés, par exemple après une électrophorèse en gel d'agarose ou de polyacrylamide, ou après une technique chromatographique comme la filtration sur gel
ou la chromatographie échangeuse d'ions, puis séquencés. La spécificité de l'amplification peut être contrôlée en utilisant comme amorce les séquences nucléotidiques de polynucléotides de l'invention comme matrice, des plasmides contenant ces séquences ou encore les produits d'amplification dérivés. Les fragments nucléotidiques amplifiés peuvent être utilisés comme réactifs dans des réactions d'hybridation afin de mettre en évidence la présence, dans un échantillon biologique, d'un acide nucléique cible de séquence complémentaire à celle desdits fragments nucléotidiques amplifiés. En règle général, selon la longueur des oligonucléotides utilisés, la température pour la réaction d'hybridation est comprise entre environ 25 et 65°C, en particulier entre 35 et 65°C dans une solution saline à une concentration d'environ 0,8 à 1 M. En ce qui concerne les sondes spécifiques, l'appariement peut s'effectuer à des températures supérieures à 50°C pour 1 à 2 mM MgCl2. La température d'hybridation d'un oligonucléotide étant calculée sur la base de 2°C par A ou T et de 4°C pour G ou C ; un oligonucléotide constitué par exemple d'une combinaison de 7 A et 5 C s'hybride à 34°C. Cette règle bien connue par l'homme du métier permet de calculer la température d'hybridation envisageable pour tous les oligonucléotides selon l'invention.

D'autres techniques d'amplification de l'acide nucléique cible peuvent être avantageusement employées comme alternative à la PCR (PCR-like) à l'aide de couple d'amorces de séquences nucléotidiques selon l'invention. Par PCR-like on entendra désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues, en général il s'agit de l'amplification de l'ADN par une polymérase ; lorsque l'échantillon d'origine est un ARN il convient préalablement d'effectuer une transcription reverse. Il existe actuellement de très nombreux procédés permettant cette amplification, comme par exemple la technique SDA (Strand Displacement Amplification) ou technique d'amplification à déplacement de brin (Walker et al., 1992), la technique TAS (Transcription-based Amplification System) décrite par Kwoh et al. en 1989, la technique 3SR (Self-Sustained Sequence Replication) décrite par Guatelli et al. en 1990, la technique NASBA (Nucleic Acid Sequence Based Amplification) décrite par Kievitis et al. en 1991, la technique TMA (Transcription Mediated Amplification), la technique LCR (Ligase Chain Reaction) décrite par Landegren et al. en 1988 et perfectionnée par Barany et al. en 1991, qui emploie une ligase thermostable, la technique de RCR (Repair Chain Reaction) décrite par Segev en 1992, la technique CPR (Cycling Probe Reaction) décrite par Duck et al. en 1990, la technique d'amplification à la Q-béta-réplicase décrite par Miele et al. en 1983 et perfectionnée notamment par Chu et al. en 1986 et Lizardi et al. en 1988, puis par Burg et al. ainsi que par Stone et al. en 1996.

Dans le cas où le polynucléotide cible à détecter est un ARNm, on utilisera avantageusement, préalablement à la mise en oeuvre d'une réaction d'amplification à l'aide des amorces selon l'invention ou à la mise en oeuvre d'un procédé de détection à l'aide des sondes de l'invention, une enzyme de type transcriptase reverse afin d'obtenir un ADNc à partir de l'ARNm contenu dans l'échantillon biologique. L'ADNc obtenu servira alors de cible pour les amorces ou les sondes mises en oeuvre dans le procédé d'amplification ou de détection selon l'invention. Comme indiqué précédemment, des sondes ou armorces pouvant discriminer entre hCTL1a, hCTL1b et hCTL2 sont particulièrement visées par l'invention.

L'invention porte également sur un kit de diagnostic **caractérisé en ce qu**'il permet de mettre en oeuvre le procédé décrit précédemment.

L'invention se rapporte également à l'utilisation de CTL4 dont la séquence peptidique et nucléotidique est disponible dans genbak sous le numéro d'accession AF1347726 et numéro GI : 4529886 (séquence intitulée NG22) dans le cadre des procédés explicités dans la description.

Un aspect complémentaire de l'invention réside en un procédé de criblage de composés susceptibles de modifier l'activité d'un polypeptide décrit ci-dessus. Ce procédé peut se **caractériser en ce qu**'il comprend les étapes suivantes :
a) expression dudit polypeptide dans une cellule hôte en utilisant un vecteur selon l'invention,
b) incubation de la cellule hôte obtenue à l'étape a) avec la choline et/ou un analogue de la choline marqué avec un isotope, notamment avec des analogues de choline tels que HC-3, HC-15, la d-tubocurarine, l'oxotremorine ou la carbamyl-b-méthylcholine et au moins un composé susceptible de modifier l'activité dudit polypeptide,
c) détection de l'incorporation de choline et/ou analyse de la quantité d'acétylcholine produite.

Bien entendu toute variante ou toute autre technique permettant d'identifier et de sélectionner des composés susceptibles de modifier l'activité des protéines CTL est un objet de l'invention. En ce sens, l'utilisation des gènes et protéines CTL pour identifier et sélectionner des composés susceptibles de modifier leur activité est particulièrement visée.

Ce procédé permet l'identification d'un composé capable de restaurer l'activité d'un polypeptide comportant au moins une mutation identifiée grâce au procédé précédemment évoqué. Il permet aussi l'identification d'un composé capable d'inhiber l'activité d'un polypeptide selon l'invention.

On peut illustrer ce procédé de criblage permettant l'identification de composés agissant sur l'activité des CTL par les exemples suivants qui pourront être mises en oeuvre lors d'expériences de routine par l'homme du métier (O'Regan S. Binding of [3H]Hemicholinium-3 to the high affinity choline transporter in Electric Organ Synaptosomal Membranes, J. ofNeurochemistry, 1988, Vol 51, N°6, 1682 :1687) :

Le ligand [3H]hémicholinium (HC-3) peut servir de marqueur d'activité du système de transport de la choline à affinité élevée étroitement associé à la synthèse de l'acétylcholine (ACh) dans les terminaisons nerveuses cholinergiques (Kuhar et Murrin, 1978 ; Jope ; 1979). Il permet en effet la caractérisation et la localisation des sites de liaisons de [3H]HC-3 dans les membranes de cellules nerveuses de rat (Vickroy et al., 1984b ; Sandberg et Coyle, 1985 ; Chatterjee et al., 1987) et le suivi des modifications qui interviennent après des lésions (Vickroy et al., 1984a), après divers traitements par des composés, notamment par des médicaments (Swann et al., 1986 ; Lowenstein et Coyle, 1986), ou en conditions de dépolarisation (Saltarelli et al., 1987) connues pour modifier d'autres paramètres cholinergiques présynaptiques.

Ainsi, dans le cadre de l'invention, il est possible de mesurer l'effet de composés sur l'activité des polypeptides CTL dans le transport de la choline dans les synaptosomes.

A cet effet, On peut mesurer la capture de la choline dans des fractions aliquotes de synaptosomes contenant de la [3H]choline (15 Ci/mmol, CEA, Saclay, France). On ajoute de la choline non marquée pour la détermination des paramètres de transport. A la fin de la période d'incubation, on centrifuge et on recupère le culot dans 50 µl de Triton X-100 à 10%. On a rince les tubes une fois et on transfère les échantillons et les liquides de rinçage dans des flacons de comptage remplis de 5 ml de liquide de scintillation Ready Protein (Beckman, Palo Alto, CA., E.U.A.). On détermine la radioactivité en utilisant un compteur du type LS3801 Beckman par exemple. Les paramètres cinétiques du transport de la choline dans la gamme d'affinité élevée sont estimés en utilisant une analyse de régression des moindres carrés non linéaire (Jolivet, 1982) pour ajuster une fonction Michaelis-Menten simple aux données expérimentales, ou des représentations de Lineweaver-Burk et Scatchard avec un programme de régression linéaire. On utilise des courbes log-logit pour déterminer les valeurs d'IC50 pour la choline (Sigma, St. Louis, MO, E.U.A.), des analogues de choline, HC-3, HC-15 (Aldrich, Steinheim, Allemagne), la d-tubocurarine (Serva, Heidelberg, Allemagne), l'oxotremorine (Sigma), et la carbamyl-b-méthylcholine (Sigma).

On peut également mesuré la liaison de HC-3 aux membranes. On utilise une technique de centrifugation rapide pour séparer le ligand lié du ligand libre dans ce procédé afin de réduire au minimum la perte de ligand lié et faciliter les comparaisons entre les différentes préparations. On mélange des fractions aliquotes (25 µl) de membranes dans du tampon Tris (10 mM, pH 8,0) avec 75 µl de NaCl 0,4 M, glycylglycine 10 mM, pH 7,1, pour déterminer la liaison totale, ou bien de LiCl 0,4 M, glycylglycine 10 mM, pH 7,1, pour déterminer la liaison indépendante du Na ; dans les deux cas, la concentration de sel finale est de 300 mM à pH 7,4, similaire à celle du milieu de liaison recommandé par Sandberg and Coyle (1985). L'équilibre est atteint au bout de 5 min d'incubation et la quantité de [3H]HC-3 liée est proportionnelle à la quantité de membranes ajoutées dans l'intervalle allant de 10 à 100 µg de protéines. Généralement, on incube des membranes contenant environ 40 µg de protéines à température ambiante (20-22°C) pendant 15 min avec 10 nM de [3H]HC-3 (132,8 Ci/mmol, New England Nuclear, Boston, MA, E.U.A.) dans des tubes airfuge ultratransparents (Beckman). On centrifuge ensuite les échantillons pendant 5 min, dans un airfuge Beckman à 100 psi (55 000 gmax.). On préleve les surnageants et on essuie les côtés et les fonds des tubes sans perturber les culots. On remet ensuite les culots en suspension dans 50 µl de Triton X-100 10%, puis on les transfère dans des flacons de comptage, avec 50 µl de solution aqueuse de rinçage, pour la détermination de la radioactivité. On détermine ainsi les paramètres de liaison en utilisant des membranes synaptosomales totales et un intervalle de concentration de [3H]HC-3 (5-120 nM), et on effectue une analyse de régression non linéaire pour ajuster la fonction B = (Bmax x F)/(KD + F) aux données expérimentales (où B est le ligand lié et F est le ligand libre), après soustraction de la liaison indépendante de Na ou en utilisant des représentations de Scatchard. Dans certains cas, on peut mesurer les valeurs de blanc en présence de 100 µM de choline non marquée.

Le nombre apparent de rotations pour le transporteur de choline peut être calculé en divisant le Vmax pour le transport de choline d'affinité élevée par le Bmax pour les sites de liaisons d'HC-3. On détermine les deux paramètres en utilisant un matériau provenant de la même préparation de synaptosomes. On normalise les données par rapport à la récupération d'AChE dans les culots finaux d'échantillons traités en parallèle, car la teneur en protéines des synaptosomes et celle des membranes synaptosomales diffèrent en raison de la contribution des protéines cytoplasmiques à la teneur en protéines

L'invention a également pour objet une composition comprenant ledit composé ou un vecteur précédemment explicité et un véhicule pharmaceutiquement acceptable. Il existe d'ailleurs une corrélation étroite entre la maladie d'Alzheimer et la quantité d'acétylcholine transférase (Baskin D. S et al Arch Neurol, 1999, Sep ; 56(9) 1121 :1123) ou l'action autoinhibitrice de l'acétylcholine endogène dans le cerveau de patients (Albrecht C et al, Exp brain Res 1999 128(3) 383 :389, démontrant l'implication du métabolisme et du transport de la choline dans cette maladie.

Ainsi, on a isolé un clone d'une librairie d'expression réalisée à partir de l'ADN du lobe électrique de torpille, ledit clone étant capable de supprimer une mutation du transport de choline dans la levure. On trouve des niveaux élevés d'expression de l'homologue chez le rat, rCTL1, dans les neurones moteurs et les oligodendrocytes, mais de plus faibles niveaux d'expression apparaissent également dans des populations neuronales dispersées dans le cerveau. Ainsi, la répartition des rCTL1 dans le SNC n'est pas seulement limitée à celle qu'on attend pour une protéine cholinergique. En outre, un ARNm de 5 kb est associé à une forte expression dans la couche cellulaire de la muqueuse du colon. Les neurones cholinergiques sont particulièrement sensibles aux modifications dans le métabolisme de la choline car ils ont besoin de la choline pour la synthèse tant de la membrane que du neurotransmetteur (25, 26). Les protéines CTL peuvent donc fournir la choline pour la synthèse des composants de la membrane et/ou être impliquées dans la synthèse de l'acétylcholine. L'expression de tCTL1 et rCTL1 sous la forme de séquences codantes complètes dans des oocytes et dans diverses cellules cultivées, montre des modifications significatives dans l'absorption de choline.

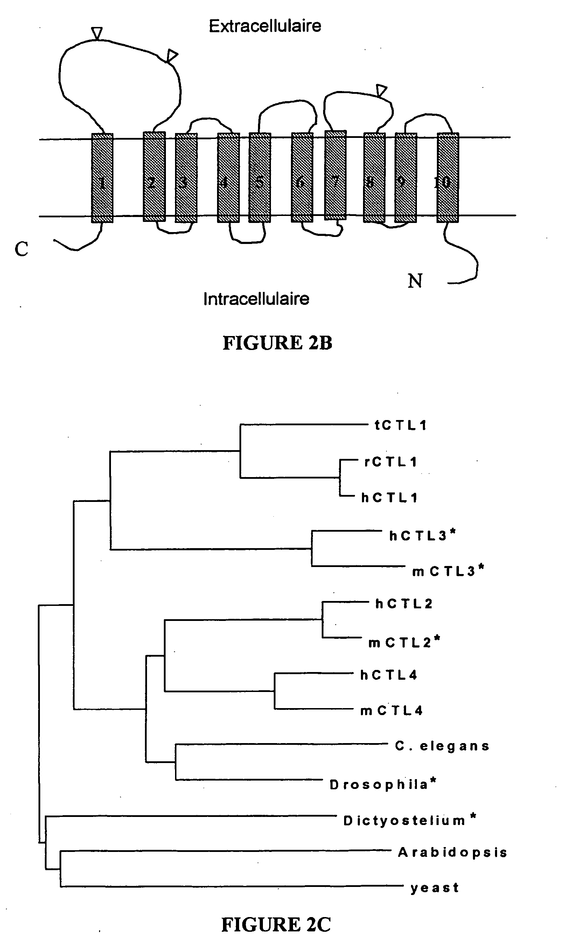

La famille CTL se caractérise par 10 domaines transmembranaires putatifs et 11 cystéines hautement conservées. Ces protéines partagent une homologie de structure limitée avec les transporteurs, et en particulier avec les domaine transmembranaires TMD 2 et 3 (voir figure 2B). Les plantes et les animaux simples, comme C. elegans, n'ont qu'un gène de cette nature (figure 2C). En revanche, il est clair que les êtres humains et les souris ont trois ou probablement quatre gènes homologues différents situés dans des positions chromosomiques où l'on retrouve de nombreux gènes dupliqués. La localisation chromosomique de hCTL1 au voisinage de D9S299 en 9q31.2 place ce gène à proximité étroite des loci responsables des maladies génétiques que sont la dysautonomie familiale et la maladie de Tangier. La forte expression de rCTL1 dans les neurones moteurs, sensitifs et les oligodendrocytes, ainsi que le lien fonctionnel avec l'absorption de choline en font un candidat prometteur pour le site de mutation provoquant la dysautonomie familiale, maladie qui inclut un composant cholinergique périphérique (27) avec des manifestations tant autonomes que motrices à la naissance, et une démyélinisation progressive du SNC chez les adultes (28). Les efforts actuels pour affiner la localisation génomique de hCTL1 combinés au récent rétrécissement de la gamme des marqueurs avoisinants le locus associé à la dysautonomie fonctionnel (29) indiquent que la région exonique de hCTL1 est proche, mais proximale, du site de mutation, laissant la possibilité d'une permutation dans le domaine régulatoire en 5'.

La mutation de la maladie de Tangier est moins bien localisée et hCTL est dans l'intervalle des marqueurs associés à cette maladie. Dans la maladie de Tangier, des niveaux réduits de lipoprotéines haute densité aboutissent à un faible niveau de cholestérol circulant avec une neuropathie récurrente et un stockage lipidique intestinal (30). On sait que la choline, via la phosphatidylcholine, intervient dans la production de lipoprotéines haute densité. En ce sens, il a été montré que la lécithine, un phospholipide synthétisé à partir de la choline, augmente la sécrétion biliaire de lipoprotéines haute densité (31).

L'identification de cette nouvelle famille de protéines chez l'homme ouvre donc la voie au développement d'outils pharmacologiques qui pourraient être utiles pour le traitement de diverses maladies liées aux perturbations des fonctions de CTL, en particulier au niveau des cellules telles que les cellules du tractus intestinales, les cellules nerveuses, notamment les motoneurones, les neurones sensitifs, les neurones du noyau dorsale de la moelle épinière et les oligodendrocytes.

On se référera aux légendes des figures présentées ci-après pour la suite de la description.

### Légendes

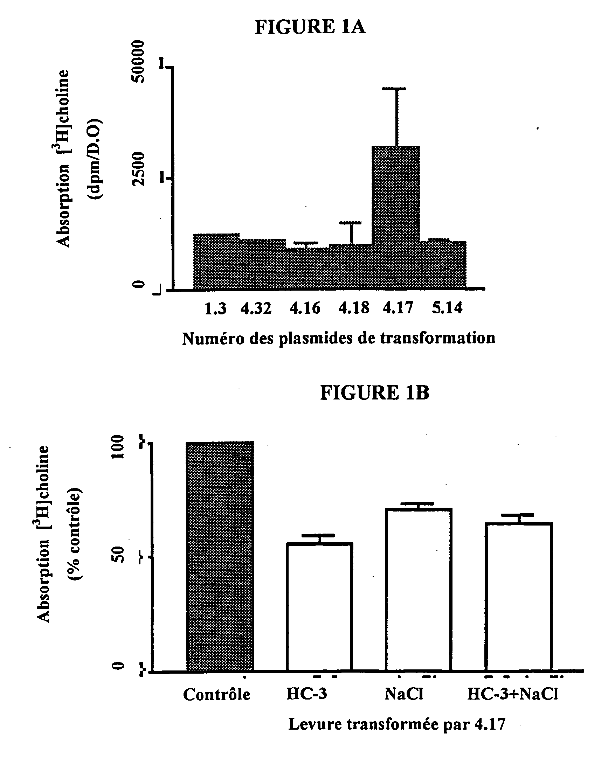

**Figure 1** **: Suppression des mutations du transport de choline chez la levure par expression hétérologue d'ADNc du lobe électrique.**
   **(A)** On a transformé des levure mutantes avec des plasmides individuels isolés à partir de colonies présentant une croissance dépendant de la choline dans des conditions sélectives. On a incubé la levure transformée à 30°C pendant 30 minutes avec de la [3H]-choline 25 nM. On a mesuré l'absorption de choline saturable sous la forme de la différence d'absorption en présence et en l'absence de choline froide 1 mM, et on normalise pour tenir compte de la croissance (DO600) ; 4.16 est un plasmide sans insert et on l'a utilisé comme témoin. Seul 4.17 a la capacité d'induire l'absorption de choline (moyenne ( écart-type pour 3-5 expériences indépendantes).
   **(B)** Caractérisation de l'absorption de choline par la levure mutante transformée par 4.17. On a inhibé l'absorption de choline dans une mesure similaire par HC-3 1 µM, par l'addition de NaCl 100 mM, et par l'addition de HC-3 comme de NaCl (moyenne ( écart-type pour 5 expériences indépendantes).

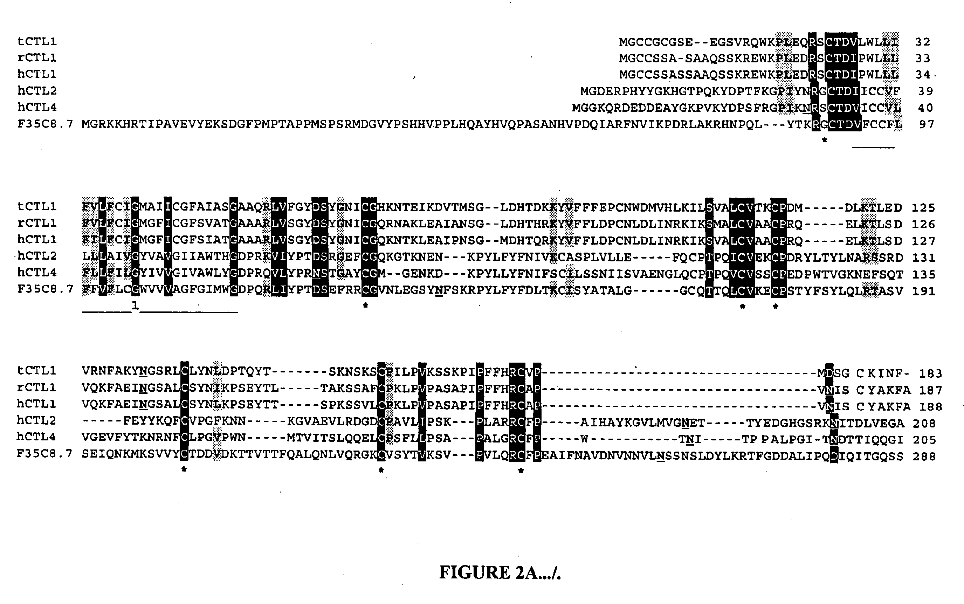

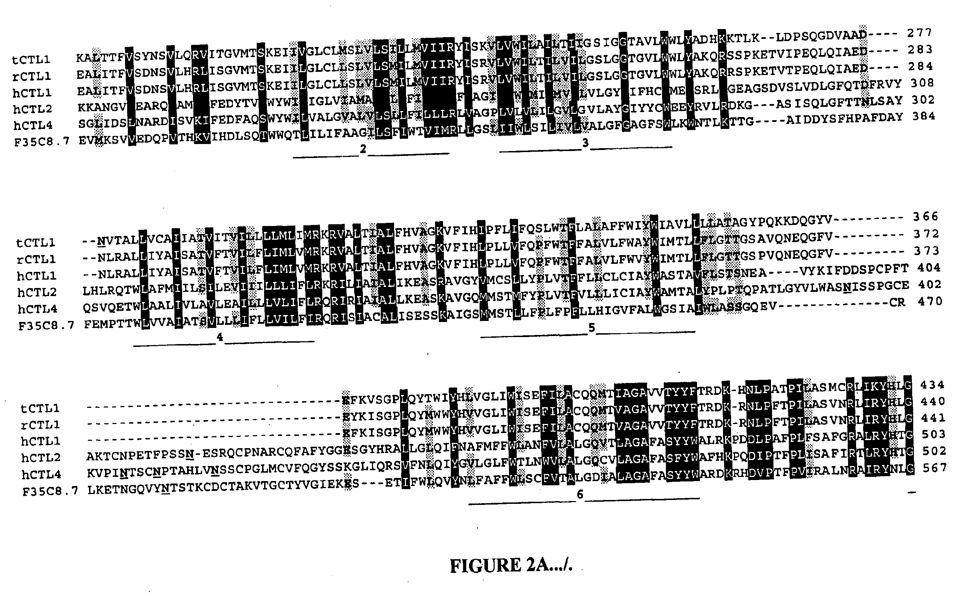

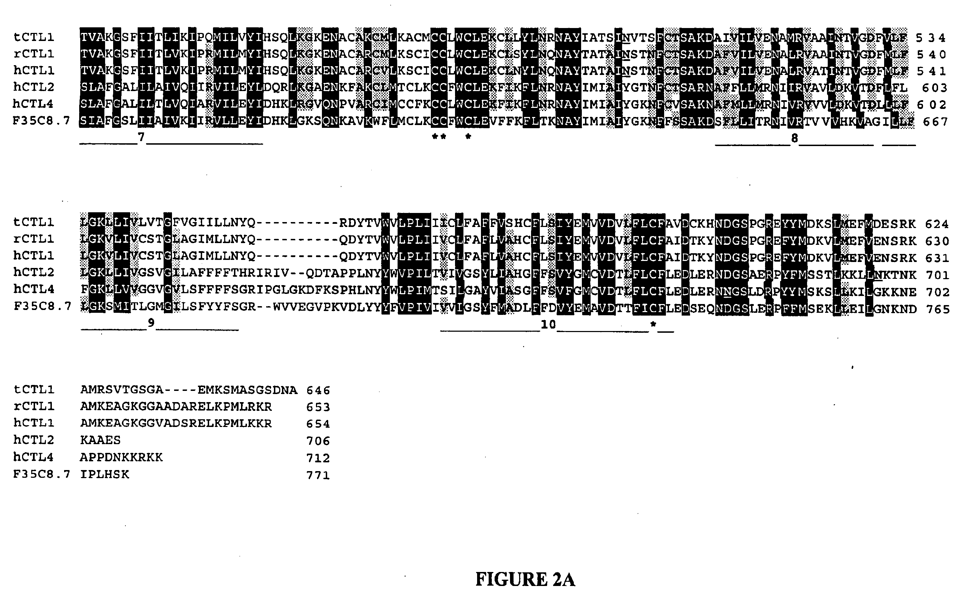

**Figure 2** **: Alignements des séquences des protéines CTL et modèle de leur topologie membranaire.**
   **(A)** Alignement de séquences d'acides aminés de CTL1 provenant de torpille (tCTL1), du rat (rCTL1) et des humains (hCTL1) avec deux protéines homologues, hCTL2 et hCTL4, et la protéine homologue de C.elegans unique, F35C8.7. Un hachurage noir indique 100 % de conservation et un hachurage gris clair 80 % de conservation (Blosum 62). Les segments d'acides aminés hydrophobes qui peuvent former les domaines transmembranaires (TMD) sont indiqués sous les alignements et numérotés. Les sites de N-glycosylation potentiels sont soulignés dans chaque séquence. Les cystéines conservées sont marquées par des astérisques sous l'alignement.
   **(B)** Modèle structural de CTL1.
   **(C)** Dendrogramme de protéines CTL et des protéines apparentées obtenu en utilisant ClustalW. On n'a pas trouvé d'homologues chez les procaryotes. Les astérisques indiquent que l'on n'utilise que les séquences protéiques partielles pour l'analyse.
**Figure 3** **: Analyse Northern de l'ARNm de rCTL1 dans des tissus de rat adultes.**
   On a hybridé l'ARN poly(A)+ (2 µg) provenant des tissus spécifiés avec une sonde pour rCTL1 et on l'a exposé pendant 7 heures. La partie basse montre l'hybridation avec une sonde de (-actine.
**Figure 4** **: Répartition de rCTL1 dans des tissus de rat adultes par ISH.**
   **(A)** Cette figure montre un marquage intense avec la sonde d'ARNc de rCTL1 antisens dans les cellules dispersées présentes à une plus haute densité dans le corps calleux (CC) que dans les cellules localisées dans la matière grise, ainsi que dans les neurones hippocampiques de la corne d'Ammon (CA1-CA3) et du corps godronné (DG).
   **(B)** Un plus fort grossissement des cellules marquées dans le CC montre des chaînes de cellules, ce qui suggère le marquage d'oligodendrocytes (têtes de flèches).
   **(C)** Une section frontale de la moelle épinière cervicale montre une densité élevée de petites cellules marquées présentes dans la matière blanche comme dans la matière grise, ainsi que de plus grandes cellules marquées observées dans les noyaux ventraux (têtes de flèches ; VMnF, fissures médio-ventrales).
   **(D)** La couche cellulaire constituant la muqueuse du colon (M) exprime de hauts niveaux de produits de transcription de rCTL1.
   **(E,F)** ISH double utilisant la sonde anti-sens d'ARNc de rCTL1 marquée à la digoxigénine **(E)** et une ribosonde antisens de choline acétyltransférase marquée à la fluorescéine **(F)** aboutit à l'identification de grandes cellules exprimant rCTL1 sous forme de neurones moteurs (têtes de flèches). Les cryosections hybridées avec des sondes sens marquées à la digoxigénine et marquées à la fluorescéine ne présentent pas de signal significatif.

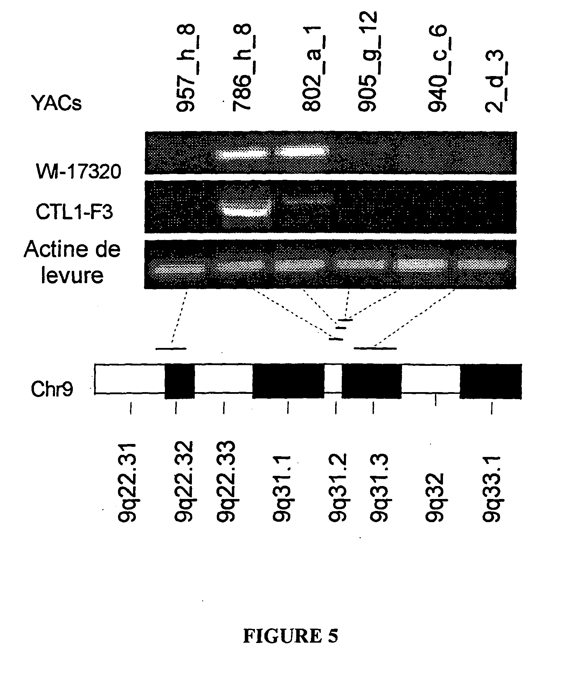

**Figure 5** **: Co-localisation de WI-17320 et hCTL-F3 à des YAC dont la carte est établie en 9q31.2.**
   Parmi les 6 YAC testés, seuls les YAC 786-h-8 et 802-a-1 sont trouvés positifs pour le marqueur WI-17320 par PCR ; les mêmes YAC sont également positifs pour un marqueur, CTLI-F3, pour la région de codage en 5' d'hCTL1. On sait également que ces deux YAC portent le marqueur génétique D9S299. On a utilisé l'actine de levure comme témoin positif La localisation des YAC sur le chromosome 9 (lignes pointillées menant aux barres) se fonde sur la représentation de cartes GDB.

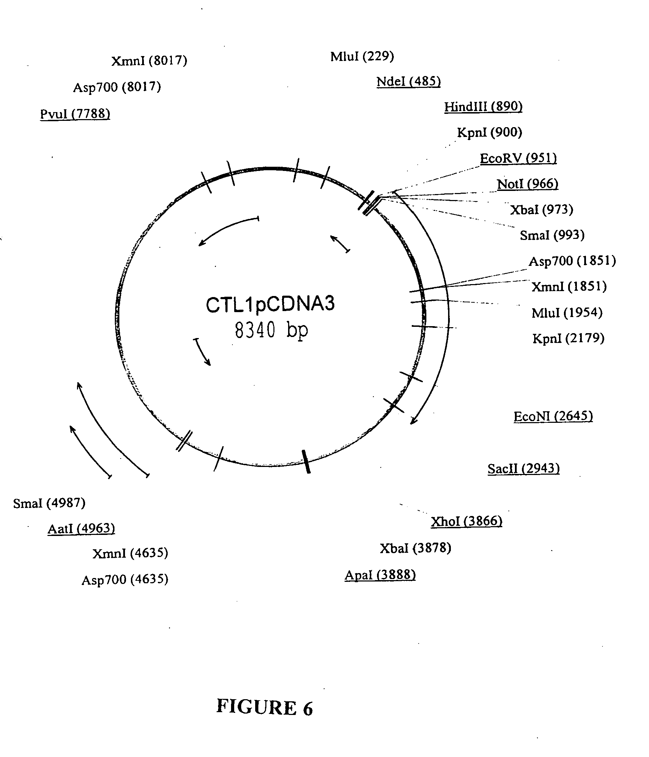

**Figure 6** **: Plasmide utilisé pour les expériences de transformation.**
   Ce plasmide a été construit à partir du vecteur d'expression chez les mammifères pcDNA3.

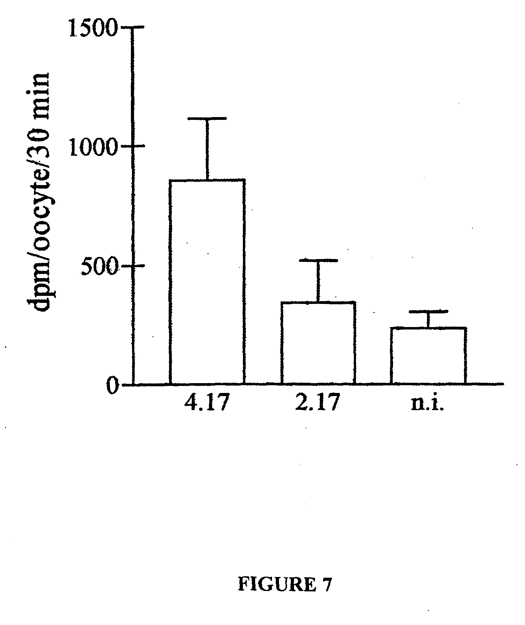

**Figure 7** **: Absorption de choline sensible à HC-3**
   Le clone 4.17 (tCTL1) a été transcrit et injecté dans des oocytes de Xenopus afin de tester l'absorption de choline en condition physiologique. Il faut noter que le niveau élevé d'absorption de choline endogène rend difficile l'observation des modifications d'absorption induite. Toutefois, l'expression de tCTL1 provoque une augmentation par un facteur 3 de l'absorption de choline sensible à HC-3 en présence de 88mM NaCl.

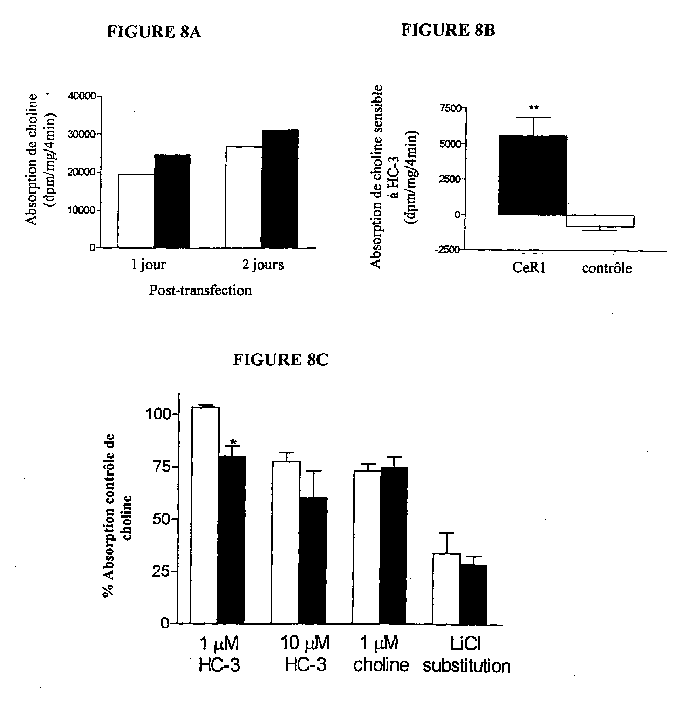

**Figure 8** **: Transfection temporaire de cellules C6 glioma par le vecteur CTL1pcDNA3 décrit à la** **figure 6****.**
   48 heures après transfection, on a observé seulement une augmentation marginale d'absorption totale de choline par rapport au cellules transfectées avec un plasmide vide (contrôle négatif). Cependant, seules les cellules C6- CTL1pcDNA3 montrent une inhibition de l'absorption totale de choline avec 1(m d'hémicholinium-3. Dès lors, CTL1 est impliqué dans le sensibilité à l'hémicholinium-3.

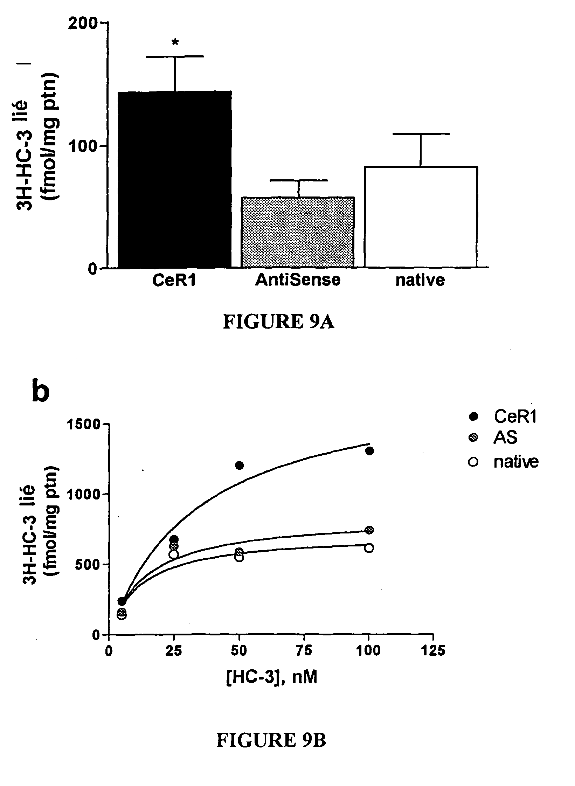

**Figure 9** **: Transfection stable de cellules C6 glioma.**
   Après sélection par antibiotiques et stabilisation, les membranes des cellules exprimant CLT1 (CeR1) retiennent 2 fois plus d'hémicholinium-3 que les cellules C6 naturelles ou que les cellules transfectées avec l'antisens de CTL1. L'expression de CTL1 est donc associée à une augmentation du nombre de protéines transporteur de choline.
**Figure 10** **: Alignements des séquences des protéines hCTL1a et hCTL1b.**
   L'épissage alternatif des transcrits du gène *CTL1* conduit à la production de deux protéines qui diffèrent dans leurs extrémités C-terminales comme indiqué sur la figure par des caractères italiques. Ces séquences pourraient jouer des rôles différents dans la régulation, l'adressage ou les interactions de ces protéines.

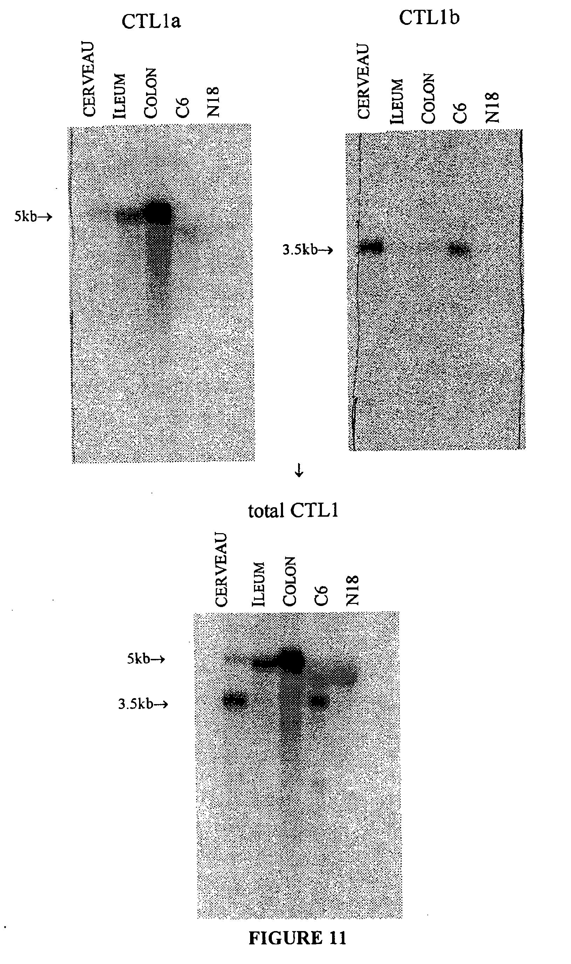

**Figure 11** **: Complémentarité d'expression des ARNm de rCTL1a et rCTL1b dans des tissus de rat adultes et des lignées cellulaires par analyse d'empreinte de Northern.**
   Des sondes spécifiques pour chaque ARNm permettent d'identifier les tissus et les cellules exprimant l'une ou l'autre des formes épissées et de caractériser leur taille. L'hybridation des mêmes empreintes avec une sonde correspondant à la séquence commune montre que ces deux formes sont les formes majoritaires de CTL1 dans les tissus de rat adulte. Parmi les lignées cellulaires examinées, le gliome C6 se distingue par une forte expression de CTL1b ainsi que par un transcrit de taille intermédiaire également détecté dans d'autres lignées telles que le neuroblastome N18. Les quantités d'ARN poly(A)⁺ déposées étaient de 2 µg pour le cerveau et le colon, et de 10 µg pour l'iléon, les lignées C6 et N18.

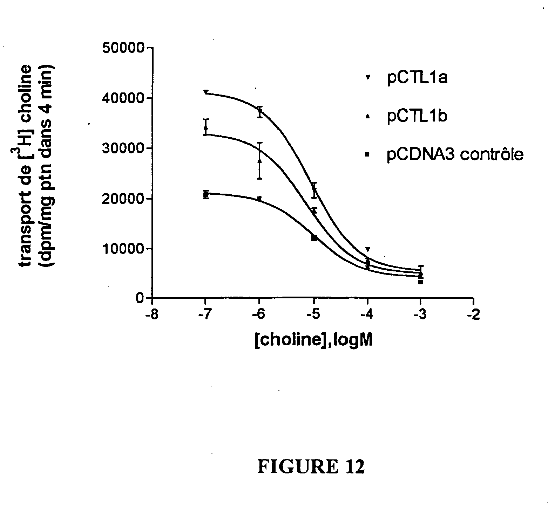

**Figure 12** **: Transport de choline par les cellules N18 après transfection transitoire par les vecteurs rCTL1a/pcDNA3, rCTL1b/pcDNA3 et pcDNA3.**
   24 heures après transfection, on observe une capture de choline plus importante par les cellules transfectées par les plasmides recombinants que par les cellules transfectées avec le plasmide vide. Dans les trois cas, la capture de choline tritiée est inhibée d'environ 50% en présence de 10µM de choline froide ou de 10 µM d'hémicholinium-3 (résultat non montré).

### Exemples 1 : Complémentation de la mutation du transport de choline dans la levure.

### Matériels et méthodes

On a obtenu la souche de levure (ctr ise URA3() utilisée dans le cadre de l'invention en croisant D308-14D (ctr ise leu his4)(6), avec une souche URA3( (9). Cette souche ne croît pas dans un milieu appauvri en uracile, ni dans une teneur élevée en myoinositol, sauf si l'on dispose à la fois d'une source externe de choline et d'un mécanisme d'absorption de choline. On a préparé de l'ARN poly(A)+ à partir de lobes électriques congelés de Torpedo marmorata. On a inséré les ADNc correspondants dans le site BstXI du plasmide pFL61 (9) en utilisant la trousse de clonage d'ADNc d'Amersham. On a procédé à la transformation de la levure en utilisant le procédé LiAc/SS-DNA(PEG) (10). On a cultivé la levure transformée sur un milieu solide dans des conditions de croissance sélectives (pas d'uracile, 20 µg/ml de choline, 20 µg/ml de myoinositol) ; on a éliminé les mutants réverses comme étant capables d'une croissance en l'absence de choline. On a isolé les plasmides à partir de colonies ayant un fort phénotype de croissance dépendant de la choline, et on les a utilisés pour transformer des fractions aliquotes de levure mutante afin d'éliminer les mutants réverses ctr et les mutations de suppresseur de levure. On a retenu 5 clones, et on a testé l'absorption de choline.

Absorption de choline par des levures recombinantes :
On a transformé des fractions aliquotes de levure mutante avec des plasmides isolés individuels et, après amplification, on a déterminé l'absorption de choline dans un milieu dépourvu d'azote (11) à 30°C pendant 30 minutes avec de la [3H]choline 25 mM (0,1 µCi, 2,8 TBq/mmole, Amersham), puis on a filtré et lavé. On a estimé les blancs en ajoutant de la choline non marquée 1 mM, et on a soustrait. On a normalisé la densité de culture en mesurant la DO600.

### Résultats

La complémentation pour la croissance dépendante de la choline dans des conditions sélectives pour la levure mutante (ctr ise URA 3() transformée avec une bibliothèque d'expression de levure de lobe électrique de torpille dans pFL61 a aboutit à l'isolement d'un clone unique nommé 4.17, associé à une augmentation de l'absorption de [3H]choline saturable par la levure mutante (figure 1A). On sait que l'absorption de choline de haute affinité par les terminaisons nerveuses cholinergiques dépend du sodium et est inhibée par de faibles concentrations d'hémicolinium-3 (HC3)(1,17) ; l'absorption de [3H] choline saturable par la levure transformée en 4.17 est inhibée par HC-3 à une faible concentration (1 µM), mais l'addition de NaCl 100 mM au milieu réduit le signal, peut être du fait de l'hyperosmolarité résultante pour la levure (figure 1B). Les effets inhibiteurs d'HC-3 et l'addition hyperosmotique de NaCl ne sont pas additifs; ce qui suggère que les deux traitements inhibent le même composant de l'absorption de la choline.

Par conséquent, le clone 4.17 du lobe électrique agit comme suppresseur de la mutation du transport de la choline de la levure, et l'absorption de la choline de la levure exprimant 4.17 est sensible à HC-3 mais non dépendante du sodium, et donc ne ressemble que partiellement au transporteur de choline neuronal. Ainsi, le séquençage de l'ADNc correspondant au clone 4.17 indique qu'il code pour protéine transmembranaire tronquée de 175 aa comprenant 3 TMD avant le codon d'arrêt. Il n'est pas impossible que la protéine transmembranaire de vertébré tronquée soit plus facilement dirigée vers la membrane plasmatique de levure (18).

### Exemple 2 : Clonage et analyse de séquences orthologues et homologues de CTL1.

### Matériels et méthodes

On a isolé des ADNc de pleine longueur, suppresseurs de la mutation de transport de la choline de levure, tCTL1 et son orthologue chez le rat, rCTL1, à partir de bibliothèques de (ZAP II (Stratagene) construites à partir du lobe électrique de T. marmorata et du cerveau de rat, respectivement. On a obtenu la séquence complète d'un clone de tCTL1 de 4,4 kb et d'un clone rCTL1 de 2,9 kb en utilisant à la fois la Sequenase T7 (Amersham) et un service de séquençage externe (ESGS-Cybergene, Evry). On a attribué les domaines transmembranaires prédits (TMD) des protéines CTL sur la base de graphes d'hydropathie de Kyte et Doolittle. On a rassemblé les marqueurs de séquence exprimées homologues (EST) en utilisant le programme BLAST. On a complété les séquences pour la région de codage de l'orthologue humain hCTL1 (Unigene Acc. : Hs. 179902), et son homologue hCTL2 (Unigene Acc : Hs. 167515 et Hs. 105509) en séquençant des produits de PCR obtenus en utilisant de l'ADN provenant de la lignée cellulaire de sarcome d'Ewing, ICB 112 (12). On a disposé d'autres séquences codantes de pleine longueur comme traductions conceptuelles de séquence génomique : NG22 murines (gbAcc: AAC84166) et humaines (gbAcc: AAD21813) sont rebaptisées ici, respectivement, mCTL4 et hCTL4 ; F35C8.7 de C. elegans; YOR 161c de levure ; F20M13.200 de A. thaliana. On a procédé à l'alignement de séquences choisies en utilisant gcg:PILEUP. L'analyse EST indique des homologues de CTL1 chez Drosophila (gbAcc: AA697340), Dictyostelium (gbAcc: C24622), et un autre homologue humain que les inventeurs appellent hCTL3 (gbAcc: Aa329432) et son équivalent murin, mCTL3 (gbAcc: W64177). Les relations d'agglutination entre ces protéines sont présentées sous la forme d'un dendrogramme dessiné en utilisant ClustalW.

### Résultats :

Les séquences protéiques pour les gènes clonés (tCTL1 et rCTL1) et leur orthologue humain (hCTL1, identité à 96 % avec le rat) sont présentées dans la figure 2A. On a trouvé en outre les membres d'une famille de plusieurs protéines apparentées chez l'homme et la souris : hCLT4 (homologie à 43 % avec hCTL1) et mCTL4, à partir de traductions conceptuelles de séquences génomiques humaines et murines (voir matériels et méthodes ci-dessus) ; et hCTL2 (homologie à 43 % avec hCTL1 et homologie à 67 % avec hCTL4), pour lesquelles on a obtenu la séquence codante complète. Toutes ces séquences sont homologues à un seul gène trouvé dans le génome de C. elegans, F35C8.7 (figure 2A). Les analyses de domaine avec les bases de données BLOCKS ou PROSITE et la recherche de motif ne sont pas consensuelles, mais les transporteurs sont fréquemment cités dans les alignements avec les domaines transmembranaires TMD 2 et 3 (figure 2B). Ces protéines présentent toutes plusieurs domaines transmembranaires et on peut penser raisonnablement qu'elles traversent dix fois la membrane (figure 2B). Les autres caractéristiques majeures des protéines de cette famille sont les suivantes : une première boucle extracellulaire grande et variable entre les TMD 1 et 2, potentiellement glycosylée, avec 6 cystéines conservées ; une troisième petite boucle extracellulaire variable entre les TMD 5 et 6, glycosylée dans certaines des protéines, une région hautement conservée qui couvre les 4 derniers TMD et inclut la quatrième boucle extracellulaires qui contient trois cystéines conservées et n'est potentiellement glycosylée que dans les orthologues de CTL1 ; et les extrémités N- et C-terminales intracellulaires variables. De façon intéressante, la région conservée inclut le suppresseur, 4.17, dont le premier ATG correspond à M471 de tCTL1. Les protéines sont dépourvues d'un peptide signal clair et on s'attend qu'elles soient ciblées sur la membrane plasmatique.

Les relations entres ces séquences et d'autres séquences partielles pour une autre protéine, CTL3, chez la souris et chez l'homme, ainsi qu'avec les homologues plus distants dans d'autres eucaryotes, y compris les traductions conceptuelles à partir de génomes de plantes, sont présentées dans la figure 2C. Chez l'homme, les membres de cette famille, hCTL2 et hCTL4, ressemblent plus étroitement au gène unique de C. elegans (homologie de 51-52 %) que hCTL1 et hCTL3 (homologie de 38 %). Les deux protéines végétales ne montrent que 25 % d'homologie avec le gène de C. elegans. Prises ensemble, les informations sur la structure et les relations de ces séquences montre qu'il s'agit bien d'une nouvelle famille de protéine CTL (abréviation de protéines du type transporteuses de C. elegans).

### Exemple 4 : Analyse de l'expression de rCTL1 dans les tissus.

### Matériels et méthodes :

### a) Northern blot.

On a analysé de l'ARN poly(A)+ (2 µg) extrait de divers tissus de rat par Northern Blot en utilisant un fragment BamHI/EcoRI en 5' de rCTL1 marqué avec [32P]dCTP, et lavé dans des conditions stringentes. On a exposé pendant 7 puis 40 heures (non représenté), et on a hybridé avec une sonde (-actine comme contrôle.

### b) Hybridation in situ.

Les expériences ISH ont été effectuées comme décrit dans (14), modification du protocole de Schaeren-Wiemers et Gerlin-Moser (13). On a sous-cloné un fragment de restriction PstI de 1,6 kb dérivé de rCTL1 dans pGEM-4Z et l'a utilisé pour synthétiser des ribosondes anti-sens et sens marquées à la digoxigénine. On a mis en oeuvre le protocole ISH double (15) en utilisant une ribosonde de choline acétyltransférase marquée à la fluorescéine codant pour un fragment correspondant aux nucléotides 1-2332 (16). La ribosonde sens de rCTL1 n'a pas donné de signal spécifique, tandis que la sonde d'ARNc anti-sens a aboutit à des produits de réaction que l'on a observé dans un anneau cytoplasmique autour du noyau cellulaire.

### Résultats :

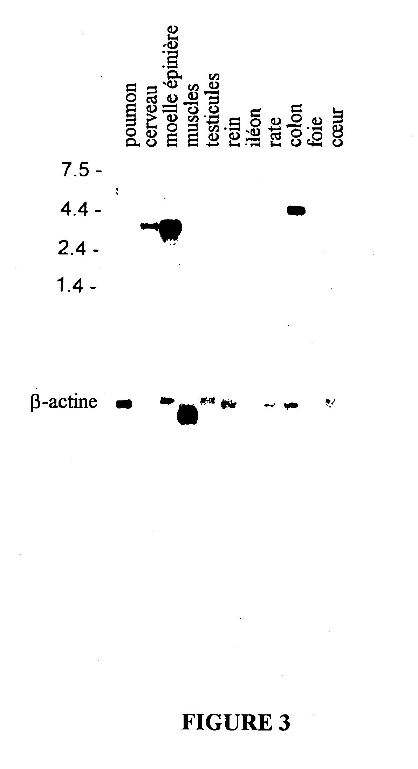

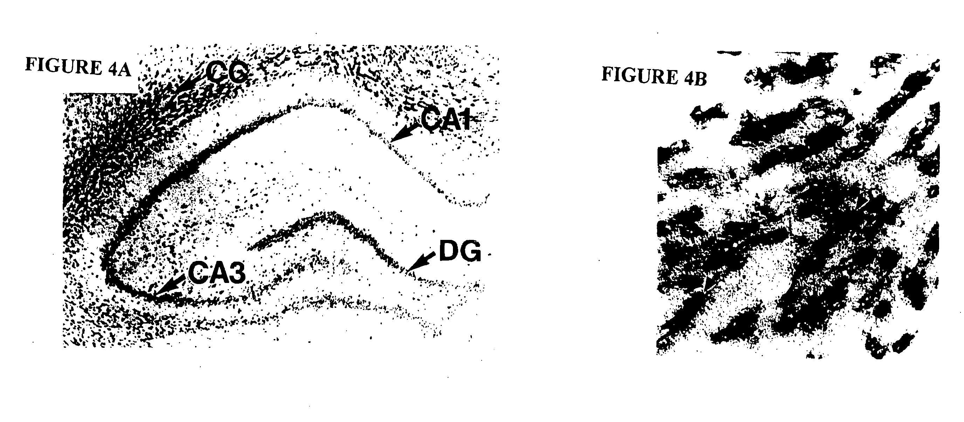

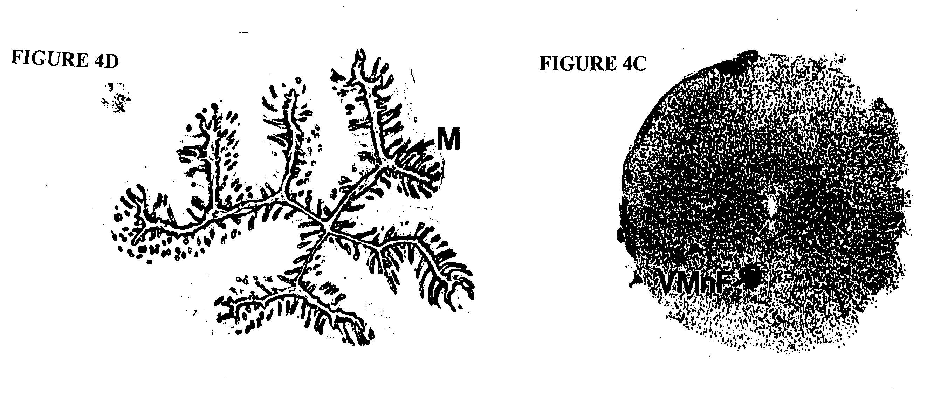

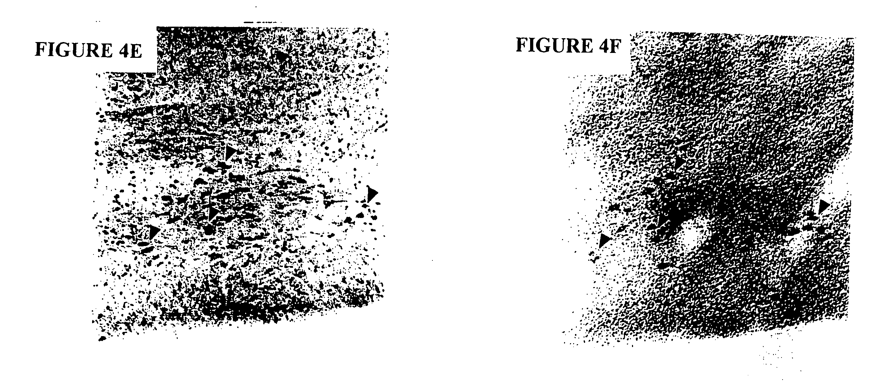

L'analyse Northern de la distribution de l'ARNm de rat avec une sonde rCTL1 présente un signal particulièrement frappant de 3,5 kb dans la moelle épinière et dans une moindre mesure dans le cerveau, tandis qu'une forme plus grande de 5 kb est présente dans le colon, dans le poumon et la moelle épinière (figure 3). La forme de 5 kb semble assez fréquente et de faible intensité (40 heures d'exposition, non représentée), tandis qu'on ne détecte pas de signal à 3,5 kb pour le foie, la rate ou l'iléon dans les mêmes conditions. Un examen plus attentif des tissus présentant une forte expression par l'ISH montre que plusieurs types de cellules expriment rCTL1 (figure 4). Dans la moelle épinière, la plus forte expression est observée dans les cellules de grande taille de la corne ventrale, qui sont identifiées comme neurones moteurs par des expériences d'ISH double utilisant, en outre, une ribosonde antisens d'ARNc de choline acétyl transférase (figure 4E, F). Les résultats montrent également une expression assez forte de produits de transcription de rCTL1 dans des neurones supposés être moteurs dans le noyau facial, ce qui suggère un rôle important dans ces neurones cholinergiques. Cependant, d'autres neurones exprimant l'ARNm de choline acétyl transférase, tels que ceux trouvés dans les zones septales ou le noyau basal, ne présentent pas de niveaux élevés d'ARNm de rCTL1. De plus, rCTL1 n'est pas un marqueur spécifique des neurones cholinergiques, même dans la moelle épinière, car une densité élevée de rCTL1 a été détectée dans des cellules dispersées dans la matière grise comme dans la matière blanche (figure 4C). Dans la matière grise, les petites cellules marquées pourraient correspondre à des oligodendrocytes et à des interneurones. Ainsi, dans le cerveau, l'expression de l'ARNm de rCTL1 est particulièrement forte dans les faisceaux de fibres myélinisées. Les résultats montrent également une densité élevée de cellules intensément marquées dans la matière blanche cérébelleuse, le tronc cérébral, le corps calleux, les fimbria hippocampiques et le pédoncule olfactif latéral. Dans ces zones, les cellules marquées apparaissent sous forme de chaîne courte (4B) comme cela a été montré pour les oligodendrocytes (19). Les expériences d'ISH double utilisant une ribosonde spécifique de la protéine de base de la myéline, un marqueur d'oligodendrocytes (19) et le rCTL1 effectuées au niveau de la matière blanche du cervelet indiquent que rCTL1 est bien exprimé dans les oligodendrocytes. rCTL1 est également exprimé à un plus faible niveau dans des neurones dispersés dans tout le cerveau, comme par exemple dans la formation hippocampique où l'on observe des produits de transcription de rCTL1 dans les cellules pyramidales de la corne d'Ammon et les cellules granulaires du corps godronné (figure A4), ainsi que dans des cellules dispersées dans toute la formation hippocampique. Ainsi, rCTL1 semble être à la fois exprimé par les neurones et les oligodendrocytes du système nerveux central (SNC).

L'expression de rCTL1 se produit également à l'extérieur du SNC, en particulier dans le colon, où le plus grand produit de transcription est fréquent. Dans le colon, on a observé des niveaux élevés d'expression dans la couche cellulaire de la muqueuse des invaginations comme dans celle du lumen (figure 4D).

### Exemple 5 : Positionnement de hCTL1 sur le chromosome 9 en utilisant des chromosomes artificiels de levure (YAC).

### Matériels et méthodes

On a lié un site marqueur de séquence (STS: WI-17320) à un EST correspondant à la région de codage en 3' de hCTL1. Les YAC dans la région indiquée, 9q22/31, proviennent du Centre d'étude du polymorphisme humain (France). On a utilisé pour l'analyse PCR la levure qui abrite les YAC. Les amorces PCR pour W1-17320 sont telles que données (gbAcc: G24229) et pour l'actine de levure (gbAcc: X61502) sont directes et réverses : CAAAATTGGCTAGAGAAACAACCG (SEQ ID N°5) et réverses : AAAGA ACAATGGCCTTATACAGG (SEQ ID N°6). Les amorces utilisées pour localiser la région de codage du gène d'hCTL1 sont directes : CATGT GGTGGTACCATGTGGTGGG (SEQ ID N°7) et réverses : CGAATAAGGCGATTT ACTGATGCC (SEQ ID N°8). Le produit PCR utilisant ces dernières amorces, CTL1-F3, est plus long que prédit par l'ADNc, soit 762 bases au lieu de 161 car il possède un intron.

### Résultats :

L'analyse des EST humains indique également que les séquences associées à hCTL1 incluent un STS, WI-17320, qui a été localisé en 9q22/31. On ne connaît pas de YAC qui portent ce marqueur, si bien que l'on a choisit 6 YAC dont on a établi la position dans ce locus pour effectuer des tests, et l'on a trouvé par une analyse PCR que deux d'entre eux, 786-H-8 et 802-A-1, sont positifs pour WI-17320. Seuls ces deux YAC sont également positifs pour un marqueur, CTL2-F3 défini pour la région de codage en 5' d'hCTL1 (figure 5). Les deux YAC portent également le marqueur génétique D9S299, ce qui indique une localisation plus précise d'hCTL1 en 9q31.2. D9S299 est juste proximal par rapport au locus de mutation de la dysautonomie familiale (MIM 223900) (20), et à l'intérieur du locus plus large donné pour la maladie de Tangier (MIM 205400) (21).

On dispose également d'une information sur la localisation chromosomique d'hCTL4 et d'hCTL2 ; on a séquencé le NG22 humain (hCTL4) en tant que partie du complexe d'histocompatibilité majeure III en 6p21.3, et la séquence d'hCTL2 assemblée inclut deux STS dont on a établi indépendamment la carte en 19p13.1. Par conséquent, CTL ressemble de nombreux gènes humains avec une duplication entre les chromosomes 1, 6, 9 et 19(22-24).

### Exemple 6 : Deux formes de CTL1 issues d'un épissage alternatif

Nous avons mis en évidence un épissage alternatif sur le gène codant pour la protéine CTL1 chez l'homme et chez le rat. Cet épissage conduit à deux protéines hCTL1a (SEQ ID No 9) et hCTL1b (SEQ ID No 3), qui ne diffèrent que par leur extrémité C-terminale. Les séquences précédant le codon stop sont respectivement MLKKR pour hCTL1b et MASGASSA pour hCTL1a (Figure 10). Ces deux formes ont une distribution tissulaire différente. Chez le rat, CTL1b est exprimé de façon prédominante dans le cerveau, alors que CTL1a est majoritairement exprimé dans l'intestin (Figure 11). A l'échelle cellulaire, des expériences d'hybridation in situ utilisant respectivement les sondes rCTL1a et rCTL1b sur des coupes de cerveau montrent que les deux transcrits sont présents dans les cellules gliales, mais seul rCTL1a est exprimé dans certaines populations neuronales. La co-localisation de rCTL1a et rCTL1b est retrouvée sur une lignée gliale, le gliome C6 (Figure 11). Après transfection dans la lignée cellulaire N18 n'exprimant que peu de CTL1 endogène (Figure 11), les plasmides contenant les ADNc codant pour rCTL1a ou pour rCTL1b induisent l'une et l'autre une augmentation de la capture de choline tritiée par les cellules (Figure 12).

Ces deux protéines sont douées d'une activité de transport de choline, et leur expression différentielle peut ouvrir la voie au développement de molécules à action sélective.

### REFERENCES

1. Yamamura, H.I. & Snyder, S.H. (1973) J.Neurochem. 21, 1355-1374.
2. Kuhar, M.J. & Murrin, L.C. (1978) J.Neurochem. 30, 15-21.
3. Jope, R.S. (1979) Brain Res. 180, 313-344.
4. Knipper, M., Kahle, C. & Breer, H. (1991) Biochim.Biophys.Acta 1065, 107-113.
5. Rylett, R.J., Walters, S.A. & Davis, W. (1996) Brain Res.Mol.Brain Res. 35, 354-358.
6. Nikawa, J., Tsukagoshi, Y. & Yamashita, S. (1986) J.Bacteriol. 166, 328-330.
7. Sentenac, H., Bonneaud, N., Minet, M., Lacroute, F., Salmon, J.M., Gaymard, F. & Grignon, C. (1992) Science 256, 663-665.
8. Nikawa, J., Hosaka, K., Tsukagoshi, Y. & Yamashita, S. (1990) J.Biol.Chem. 265, 15996-16003.
9. Minet, M., Dufour, M.E. & Lacroute, F. (1992) Plant J. 2, 417-422.
10. Gietz, R.D., Schiestl, R.H., Willems, A.R. & Woods, R.A. (1995) Yeast. 11, 355-360.
11. Hosaka, K. & Yamashita, S. (1980) J.Bacteriol. 143, 176-181.
12. O'Regan S., Diebler, M.-F., Meunier, F.-M. & Vyas S. (1995) J.Neurochem. 64, 69-76.
13. Schaeren-Wiemers, N. & Gerfin-Moser, A. (1993) Histochemistry 100, 431-440.
14. Traiffort, E., Charytoniuk, D.A., Watroba, L., Faure, H., Sales, N. & Ruat, M. (in press) Eur.J.Neurosci.
15. Arce, V., Pollock, R.A., Philippe, J.M., Pennica, D., Henderson, C.E. & deLapeyriere, O. (1998) J.Neurosci. 18, 1440-1448.
16. Brice, A., Berrard, S., Raynaud, B., Ansieau, S., Coppola, T., Weber, M.J. & Mallet, J. (1989) J.Neurosci.Res. 23, 266-273.
17. Haga, T. & Noda, H. (1973) Biochim.Biophys.Acta 291, 564-575.
18. Hogue, D.L., Ellison, M.J., Young, J.D. & Cass, C.E. (1996) J.Biol.Chem. 271, 9801-9808.
19. Shiota, C., Miura, M. & Mikoshiba, K. (1989) Brain Res.Dev.Brain Res. 45,83-94.
20. Blumenfeld, A., Slaugenhaupt, S.A., Axelrod, F.B., Lucente, D.E., Maayan, C., Liebert, C.B., Ozelius, L.J., Trofatter, J.A., Haines, J.L. & Breakefield, X.O. (1993) Nat.Genet. 4, 160-164.
21. Rust, S., Walter, M., Funke, H., von Eckardstein, A., Cullen, P., Kroes, H.Y., Hordijk, R., Geisel, J., Kastelein, J., Molhuizen, H.O., Schreiner, M., Mischke, A., Hahmann, H.W. & Assmann, G. (1998) Nat.Genet. 20, 96-98.
22. Katsanis, N., Fitzgibbon, J. & Fisher, E.C. (1996) Genomics 35, 101-108.
23. Sugaya, K., Sasanuma, S., Nohata, J., Kimura, T., Fukagawa, T., Nakamura, Y., Ando, A., Inoko, H., Ikemura, T. & Mita, K. (1997) Gene 189, 235-244.
24. Hughes, A.L. (1998) Mol.Biol.Evol. 15, 854-870.
25. Blusztajn, J.K. & Wurtman, R.J. (1983) Science 221, 614-620.
26. Wurtman, R.J. (1992) Trends.Neurosci. 15, 117-122.
27. Mittag, T.W., Mindel, J.S. & Green, J.P. (1974) Ann.N.Y.Acad.Sci. 228, 301-306.
28. Pearson, J. & Pytel, B.A. (1978) J.Neurol.Sci. 39, 47-59.
29. Blumenfeld, A., Slaugenhaupt, S.A., Liebert, C.B., Temper, V., Maayan, C., Gill, S., Lucente, D.E., Idelson, M., MacCormack, K., Monahan, M.A., Mull, J., Leyne, M., Mendillo, M., Schiripo, T., Mishori, E., Breakefield, X., Axelrod, F.B. & Gusella, J.F. (1999) Am.J.Hum.Genet. 64, 1110-1118.
30. Engel, W.K., Dorman, J.D., Levy, R.I. & Fredrickson, D.S. (1967) Arch.Neurol. 17, 1-9.
31. LeBlanc, M.J., Gavino V., Perea, A., Yousef, I.M., Levy, E. & Tuchweber, B. (1998) Biochim.Biophys.Acta 1393, 223-234.

## Revendications

1. Acide nucléique purifié ou isolé, **caractérisé en ce qu'**il comprend une séquence nucléique choisie parmi le groupe de séquences suivantes :
a) la séquence SEQ ID No. 1 ;
b) la séquence SEQ ID No. 2 ;
c) une séquence nucléique présentant un pourcentage d'identité globale d'au moins 80 % sur toute sa longueur après alignement optimal avec une séquence telle que définie en a) ou b), ladite séquence codant un polypeptide impliqué dans le métabolisme et/ou le transport de choline dans les cellules, en particulier dans les cellules nerveuses ;
d) la séquence complémentaire ou la séquence de l'ARN correspondant à une séquence telle que définie en a), b), ou c).

2. Acide nucléique purifié ou isolé selon la revendication 1, **caractérisé en ce qu'**il est constitué de la séquence SEQ ID No. 1, la séquence complémentaire ou de la séquence de l'ARN correspondant de la séquence SEQ ID No. 1.

3. Acide nucléique purifié ou isolé selon la revendication 1, **caractérisé en ce qu'**il est constitué de la séquence SEQ ID No. 2, la séquence complémentaire ou de la séquence de l'ARN correspondant de la séquence SEQ ID No. 2.

4. Acide nucléique codant pour un polypeptide sélectionné parmi hCTLIb de séquence SEQ ID N°3, hCTL1a de séquence SEQ ID N°9 et hCTL2 de séquence SEQ ID N°4.

5. Polypeptide **caractérisé en ce qu'**il comprend une séquence peptidique ayant au moins 80% d'identité globale sur toute sa longueur après alignement optimal avec une séquence sélectionnée parmi les SEQ ID N°3, 4 et 9, ledit polypeptide étant impliqué dans le métabolisme et/ou le transport de choline dans les cellules, en particulier dans les cellules nerveuses.

6. Polypeptide selon la revendication 5 ayant une séquence sélectionnée parmi les SEQ ID N°3, 4 et 9.

7. Polypeptide selon l'une des revendications 5 et 6 **caractérisé en ce que** son substrat est la choline.

8. Polypeptide selon l'une des revendications 5 à 7 **caractérisé en ce qu'**il est impliqué dans la production de l'acétylcholine et/ou dans la production des composants phospholipidiques de la membrane des cellules, notamment des cellules du tractus intestinal, des cellules nerveuses telles que les motoneurones, les neurones sensitifs, les neurones du noyau dorsal de la moelle épinière et les oligodendrocytes.

9. Vecteur comprenant une séquence selon l'une des revendications 1 à 4.

10. Vecteur selon la revendication 9 **caractérisé en ce que** ladite séquence est fusionnée à un promoteur efficace dans les cellules eucaryotes et/ou procaryotes.

11. Vecteur selon l'une des revendications 9 et 10 **caractérisé en ce qu'**il comporte en outre un gène de sélection.

12. Cellule transformée par un vecteur selon l'une des revendications 9 à 11.

13. Anticorps capable de se lier spécifiquement à un polypeptide selon la revendication 6.

14. Procédé permettant l'amplification et/ou la détection d'un acide nucléique selon l'une des revendications 1 à 4 dans un échantillon **caractérisé en ce qu'**on utilise comme amorce et/ou sonde au moins un oligonucléotide comprenant au moins 12 nucléotides consécutifs d'une séquence choisie parmi les séquences suivantes :
a) la séquence SEQ ID N°2 ;
b) une séquence nucléique présentant un pourcentage d'identité globale d'au moins 80 % sur toute sa longueur après alignement optimal avec une séquence telle que définie en a), ladite séquence codant un polypeptide impliqué dans le métabolisme et/ou le transport de choline dans les cellules, en particulier dans les cellules nerveuses ;
c) la séquence complémentaire ou la séquence de l'ARN correspondant à une séquence telle que définie en a) ou b).

15. Kit de diagnostic **caractérisé en ce qu'**il permet de mettre en oeuvre le procédé selon la revendication 14.

16. Procédé de criblage de composés susceptibles de modifier l'activité d'un polypeptide selon l'une des revendications 5 à 8 **caractérisé en ce qu'**il comprend les étapes suivantes:
a) expression dudit polypeptide dans une cellule hôte en utilisant un vecteur selon l'une des revendications 9 à 11,
b) incubation de la cellule hôte obtenue à l'étape a) avec la choline et/ou un analogue de la choline marqué avec un isotope, notamment avec des analogues de choline tels que HC-3, HC-15, la d-tubocurarine, l'oxotremorine ou la carbamyl-b-méthylcholine et au moins un composé susceptible de modifier l'activité dudit polypeptide,
c) détection de l'incorporation de choline et/ou analyse de la quantité d'acétylcholine produite.

17. Procédé selon la revendication 16 permettant l'identification d'un composé capable de restaurer l'activité d'un polypeptide selon l'une des revendications 5 à 8.

18. Procédé selon la revendication 16 permettant l'identification d'un composé capable d'inhiber l'activité d'un polypeptide selon l'une des revendications 5 à 8.

19. Composition comprenant un vecteur selon l'une des revendications 9 à 11 et un véhicule pharmaceutiquement acceptable.

## Claims

1. Purified or isolated nucleic acid, **characterized in that** it comprises a nucleic sequence chosen from the group having the following sequences:
a) the sequence SEQ ID No. 1;
b) the sequence SEQ ID No. 2;
c) a nucleic sequence exhibiting a percentage of overall identity of at least 80% over its entire length after optimal alignment with a sequence as defined in a) or b), said sequence coding for a polypeptide involved in the metabolism and/or transport of choline in the cells, in particular in the nervous cells;
d) the complementary sequence or the RNA sequence corresponding to a sequence as defined in a), b) or c).

2. Purified or isolated nucleic acid according to Claim 1, **characterized in that** it consists of the sequence SEQ ID No. 1, the complementary sequence or of the corresponding RNA sequence of the sequence SEQ ID No. 1.

3. Purified or isolated nucleic acid according to Claim 1, **characterized in that** it consists of the sequence SEQ ID No. 2, the complementary sequence or of the corresponding RNA sequence of the sequence SEQ ID No. 2.

4. Nucleic acid encoding a polypeptide selected from hCTL1b having the sequence SEQ ID No. 3, hCTL1a having the sequence SEQ ID No. 9 and hCTL2 having the sequence SEQ ID No. 4.

5. Polypeptide, **characterized in that** it comprises a peptide sequence having at least 80% overall identity over its entire length after optimal alignment with a sequence selected from SEQ ID No. 3, 4 and 9, said polypeptide being involved in the metabolism and/or the transport of choline in the cells, in particular in the nervous cells.

6. Polypeptide according to Claim 5, having a sequence selected from SEQ ID No. 3, 4 and 9.

7. Polypeptide according to either of Claims 5 and 6, **characterized in that** its substrate is choline.

8. Polypeptide according to one of Claims 5 to 7, **characterized in that** it is involved in the production of acetylcholine and/or in the production of the phospholipid components of the membrane of cells, in particular of intestinal tract cells, nervous cells such as motoneurons, sensitive neurons, neurons of the nucleus dorsalis of the spinal cord and oligodendrocytes.

9. Vector comprising a sequence according to one of Claims 1 to 4.

10. Vector according to Claim 9, **characterized in that** said sequence is fused with a promoter which is effective in eukaryotic and/or prokaryotic cells.

11. Vector according to either of Claims 9 and 10, **characterized in that** it additionally comprises a selectable gene.

12. Cell transformed with a vector according to one of Claims 9 to 11.

13. Antibody capable of binding specifically to a polypeptide according to Claim 6.

14. Method allowing the amplification and/or detection of a nucleic acid according to one of Claims 1 to 4 in a sample, **characterized in that** at least one oligonucleotide comprising at least 12 consecutive nucleotides of a sequence chosen from the following sequences:
a) the sequence SEQ ID No. 2;
b) a nucleic sequence exhibiting a percentage of overall identity of at least 80% over its entire length after optimal alignment with a sequence as defined in a), said sequence coding for a polypeptide involved in the metabolism and/or transport of choline in the cells, in particular in the nervous cells;
c) the complementary sequence or the RNA sequence corresponding to a sequence as defined in a) or b),
is used as primer and/or probe.

15. Diagnostic kit, **characterized in that** it makes it possible to carry out the method according to Claim 14.

16. Method for screening compounds capable of modifying the activity of a polypeptide according to one of Claims 5 to 8, **characterized in that** it comprises the following steps:
a) expression of said polypeptide in a host cell using a vector according to one of Claims 9 to 11,
b) incubation of the host cell obtained in step a) with choline and/or a choline analog labeled with an isotope, in particular with choline analogs such as HC-3, HC-15, d-tubocurarine, oxotremorine or carbamyl-b-methylcholine and at least one compound capable of modifying the activity of said polypeptide,
c) detection of the incorporation of choline and/or analysis of the quantity of acetylcholine produced.

17. Method according to Claim 16, allowing the identification of a compound capable of restoring the activity of a polypeptide according to one of Claims 5 to 8.

18. Method according to Claim 16, allowing the identification of a compound capable of inhibiting the activity of a polypeptide according to one of Claims 5 to 8.

19. Composition comprising a vector according to one of Claims 9 to 11 and a pharmaceutically acceptable vehicle.

## Patentansprüche

1. Gereinigte oder isolierte Nukleinsäure, **dadurch gekennzeichnet, dass** sie eine Nukleinsäuresequenz umfasst, die ausgewählt ist aus der Gruppe von folgenden Sequenzen:
a) der Sequenz SEQ ID Nr. 1;
b) der Sequenz SEQ ID Nr. 2;
c) einer Nukleinsäuresequenz, welche einen Gesamt-Identitätsprozentsatz von mindestens 80% über ihre gesamte Länge nach optimalem Alignment mit einer Sequenz, wie in a) oder b) definiert, aufweist, wobei die Sequenz ein Polypeptid kodiert, das an dem Stoffwechsel und/oder dem Transport von Cholin in den bzw. in die Zellen, insbesondere in den bzw. in die Nervenzellen, beteiligt ist;
d) der komplementären Sequenz oder der Sequenz der RNA, welche einer Sequenz, wie in a), b) oder c) definiert, entspricht.

2. Gereinigte oder isolierte Nukleinsäure nach Anspruch 1, **dadurch gekennzeichnet, dass** sie aus der Sequenz SEQ ID Nr. 1, der komplementären Sequenz oder der Sequenz der RNA, welche der Sequenz SEQ ID Nr. 1 entspricht, besteht.

3. Gereinigte oder isolierte Nukleinsäure nach Anspruch 1, **dadurch gekennzeichnet, dass** sie aus der Sequenz SEQ ID Nr. 2, der komplementären Sequenz oder der Sequenz der RNA, welche der Sequenz SEQ ID Nr. 2 entspricht, besteht.

4. Nukleinsäure, die ein Polypeptid kodiert, das aus hCTL1b mit der Sequenz SEQ ID Nr. 3, hCTL1a mit der Sequenz SEQ ID Nr. 9 und hCTL2 mit der Sequenz SEQ ID Nr. 4 ausgewählt ist.

5. Polypeptid, **dadurch gekennzeichnet, dass** es eine Peptidsequenz mit mindestens 80% Gesamt-Identität über ihre gesamte Länge nach optimalem Alignment mit einer aus den SEQ ID Nr. 3, 4 und 9 ausgewählten Sequenz umfasst, wobei das Polypeptid an dem Stoffwechsel und/oder Transport von Cholin in den bzw. in die Zellen, insbesondere in den bzw. in die Nervenzellen, beteiligt ist.

6. Polypeptid nach Anspruch 5 mit einer Sequenz, die aus den SEQ ID Nr. 3, 4 und 9 ausgewählt ist.

7. Polypeptid nach einem der Ansprüche 5 und 6, **dadurch gekennzeichnet, dass** sein Substrat Cholin ist.

8. Polypeptid nach einem der Ansprüche 5 bis 7, **dadurch gekennzeichnet, dass** es an der Produktion von Acetylcholin und/oder an der Produktion der Phospholipid-Bestandteile der Membran von Zellen, insbesondere der Zellen des Darmtrakts, der Nervenzellen, wie der Motoneuronen, der sensiblen Neuronen, der Neuronen des Nucleus dorsalis des Rückenmarks und der Oligodendrozyten, beteiligt ist.

9. Vektor, welcher eine Sequenz nach einem der Ansprüche 1 bis 4 umfasst.

10. Vektor nach Anspruch 9, **dadurch gekennzeichnet, dass** die Sequenz an einen Promotor, der in den eukaryotischen und/oder prokaryotischen Zellen wirksam ist, fusioniert ist.

11. Vektor nach einem der Ansprüche 9 und 10, **dadurch gekennzeichnet, dass** er außerdem ein Selektionsgen umfasst.

12. Zelle, die durch einen Vektor nach einem der Ansprüche 9 bis 11 transformiert ist.

13. Antikörper, der in der Lage ist, sich spezifisch an ein Polypeptid nach Anspruch 6 zu binden.

14. Verfahren, welches die Amplifizierung und/oder den Nachweis einer Nukleinsäure nach einem der Ansprüche 1 bis 4 in einer Probe erlaubt, **dadurch gekennzeichnet, dass** man als Primer und/oder Sonde mindestens ein Oligonukleotid einsetzt, welches mindestens 12 aufeinander folgende Nukleotide einer Sequenz umfasst, welche ausgewählt ist aus den folgenden Sequenzen:
a) der Sequenz SEQ ID Nr. 2;
b) einer Nukleinsäuresequenz, welche einen Gesamt-Identitätsprozentsatz von mindestens 80% über ihre gesamte Länge nach optimalem Alignment mit einer Sequenz, wie in a) definiert, aufweist, wobei die Sequenz ein Polypeptid kodiert, das an dem Stoffwechsel und/oder Transport von Cholin in den bzw. in die Zellen, insbesondere in den bzw. in die Nervenzellen, beteiligt ist;
c) der komplementären Sequenz oder der Sequenz der RNA, welche einer Sequenz, wie in a) oder b) definiert, entspricht.

15. Diagnose-Kit, **dadurch gekennzeichnet, dass** er erlaubt, das Verfahren nach Anspruch 14 auszuführen.

16. Verfahren zum Screenen von Verbindungen, die in der Lage sind, die Aktivität eines Polypeptids nach einem der Ansprüche 5 bis 8 zu modifizieren, **dadurch gekennzeichnet, dass** es die folgenden Schritte umfasst:
a) Expression des Polypeptids in einer Wirtszelle unter Einsatz eines Vektors nach einem der Ansprüche 9 bis 11,
b) Inkubation der in Schritt a) erhaltenen Wirtszelle mit Cholin und/oder einem Analogon von Cholin, welches mit einem Isotop markiert ist, insbesondere mit Analoga von Cholin wie HC-3, HC-15, d-Tubocurarin, Oxotremorin oder Carbamyl-b-methylcholin, und mindestens einer Verbindung, die in der Lage ist, die Aktivität des Polypeptids zu modifizieren,
c) Nachweisen des Einbaus von Cholin und/oder Analyse der hergestellten Menge von Acetylcholin.

17. Verfahren nach Anspruch 16, welches die Identifizierung einer Verbindung ermöglicht, die in der Lage ist, die Aktivität eines Polypeptids nach einem der Ansprüche 5 bis 8 wiederherzustellen.

18. Verfahren nach Anspruch 16, welches die Identifizierung einer Verbindung ermöglicht, die in der Lage ist, die Aktivität eines Polypeptids nach einem der Ansprüche 5 bis 8 zu inhibieren.

19. Zusammensetzung, welche einen Vektor nach einem der Ansprüche 9 bis 11 und einen pharmazeutisch annehmbaren Träger umfasst.


### LISTE DE SÉQUENCES

<110> Centre National de la Recherche Scientifique (CNRS)
<120> Protéines membranaires CTL (Choline Transporter Like) impliquées dans le transport de la choline
<130> D18413
<140>
   <141>
<150> FR 99 13883
   <151> 1999-11-05
<160> 9
<170> PatentIn Ver. 2.1
<210> 1
   <211> 2224
   <212> ADN
   <213> Homo sapiens
<220>
   <223> hCTL1b
<400> 1
   gctgcgcgca cgcgaccgca tccgggctcc ttcggccccg ccatgggctg ctgcagctcc 60
   gcttcctccg ccgcgcagag ctccaaacga gaatggaagc cgctggagga ccgtagctgc 120
   acagacatac catggctgct gctcttcatc ctcttctgca ttgggatggg atttatttgt 180
   ggcttttcaa tagcaacagg tgcagcagca agactagtgt caggatacga cagctatgga 240
   aatatccgtg ggcagaaaaa tacaaagttg gaagcaatac caaacagtgg catggaccac 300
   acccagcgga agtatgtatt ctttttggat ccatgcaacc tggacttgat aaaccggaag 360
   attaagtctg tagcactgtg tgtagcagcg tgtccaaggc aagaactgaa aactctgagt 420
   gatgttcaga agtttgcaga gataaatggt tcagccctat gtagctacaa cctaaagcct 480
   tctgaataca ctacatctcc aaaatcttct gttctctgcc ccaaactacc agttccagcg 540
   agtgcaccta ttccattctt ccatcgctgt gctcctgtga acatttcctg ctatgccaag 600
   tttgcagagg ccctgatcac ctttgtcagt gacaatagtg tcttacacag gctgattagt 660
   ggagtaatga ccagcaaaga aattatattg ggactttgct tgttatcact agttctatcc 720
   atgattttga tggtgataat caggtatata tcaagagtac ttgtgtggat cttaacgatt 780
   ctggtcatac tcggttcact tggaggcaca ggtgtactat ggtggctgta tgcaaagcaa 840
   agaaggtctc ccaaagaaac tgttactcct gagcagcttc agatagctga agacaatctt 900
   cgggccctcc tcatttatgc catttcagct acagtgttca cagtgatctt attcctgata 960
   atgttggtta tgcgcaaacg tgttgctctt accatcgcct tgttccacgt agctggcaag 1020
   gtcttcattc acttgccact gctagtcttc caacccttct ggactttctt tgctcttgtc 1080
   ttgttttggg tgtactggat catgacactt ctttttcttg gcactaccgg cagtcctgtt 1140
   cagaatgagc aaggctttgt ggagttcaaa atttctgggc ctctgcagta catgtggtgg 1200
   taccatgtgg tgggcctgat ttggatcagt gaatttattc tagcatgtca gcagatgaca 1260
   gtggcaggag ctgtggtaac atactatttt actagggata aaaggaattt gccatttaca 1320
   cctattttgg catcagtaaa tcgccttatt cgttaccacc taggtacggt ggcaaaagga 1380
   tctttcatta tcacattagt caaaattccg cgaatgatcc ttatgtatat tcacagtcag 1440
   ctcaaaggaa aggaaaatgc ttgtgcacga tgtgtgctga aatcttgcat ttgttgcctt 1500
   tggtgtcttg aaaagtgcct aaattattta aatcagaatg catacacagc cacagctatc 1560
   aacagcacca acttctgcac ctcagcaaag gatgcctttg tcattctggt ggagaatgct 1620
   ttgcgagtgg ctaccatcaa cacagtagga gattttatgt tattccttgg caaggtgctg 1680
   atagtctgca gcacaggttt agctgggatt atgctgctca actaccagca ggactacaca 1740
   gtatgggtgc tgcctctgat catcgtctgc ctctttgctt tcctagtcgc tcattgcttc 1800
   ctgtctattt atgaaatggt agtggatgta ttattcttgt gttttgccat tgatacaaaa 1860
   tacaatgatg ggagccctgg cagagaattc tatatggata aagtgctgat ggagtttgtg 1920
   gaaaacagta ggaaagcaat gaaagaagct ggtaagggag gcgtcgctga ttccagagag 1980
   ctaaagccga tgctgaagaa aaggtgactg gtctcatgag ccctgaagaa tgaactcaga 2040
   ggaggttgtt tacatgaggt tctcccactc accagctgtt gagagtctgc gattatgaag 2100
   agcaggatct tattacttca atgaaagcat gtaacaagtt tctcaaacca ccaacagcca 2160
   agtggatttg gtacagtgcg gctgtctaat aaataatcaa aagcatttga tagaaaaaaa 2220
   aaaa                                                              2224
<210> 2
   <211> 2808
   <212> ADN
   <213> Homo sapiens
<220>
   <223> hCTL2
<400> 2
   gcggccgccg gggctggtcg cctgcaggga tgggggacga gcggccccac tactacggga 60
   aacacggaac gccacagaag tatgatccca ctttcaaagg acccatttac aataggggct 120
   gcacggatat catatgctgt gtgttcctgc tcctggccat tgtgggctac gtggctgtag 180
   gcatcatagc ctggactcat ggagaccctc gaaaggtgat ctaccccact gatagccggg 240
   gcgagttctg cgggcagaag ggcacaaaaa acgagaacaa accctatctg ttttatttca 300
   acattgtgaa atgtgccagc cccctggttc tgctggaatt ccaatgtccc actccccaga 360
   tctgcgtgga aaaatgcccc gaccgctacc tcacgtacct gaatgctcgc agctcccggg 420
   actttgagta ctataagcag ttctgtgttc ctggcttcaa gaacaataaa ggagtggctg 480
   aggtgcttcg agatggtgac tgccctgctg tcctcatccc cagcaaaccc ttggcccgga 540
   gatgcttccc cgctatccac gcctacaagg gtgtcctgat ggtgggcaat gagacgacct 600
   atgaggatgg gcatggctcc cggaaaaaca tcacagacct ggtggagggc gccaagaaag 660
   ccaatggagt cctagaggcg cggcaactcg ccatgcgcat atttgaagat tacaccgtct 720
   cttggtactg gattatcata ggcctggtca ttgccatggc gatgagcctc ctgttcatca 780
   tcctgcttcg cttcctggct ggtattatgg tctgggtgat gatcatcatg gtgattctgg 840
   tgctgggcta cggaatattt cactgctaca tggagtactc ccgactgcgt ggtgaggccg 900
   gctctgatgt ctctttggtg gacctcggct ttcagacgga tttccgggtg tacctgcact 960
   tacggcagac ctggttggcc tttatgatca ttctgagtat ccttgaagtc attatcatct 1020
   tgctgctcat ctttctccgg aagagaattc tcatcgcgat tgcactcatc aaagaagcca 1080
   gcagggctgt gggatacgtc atgtgctcct tgctctaccc actggtcacc ttcttcttgc 1140
   tgtgcctctg catcgcctac tgggccagca ctgctgtctt cctgtccact tccaacgaag 1200
   cggtctataa gatctttgat gacagcccct gcccatttac tgcgaaaacc tgcaacccag 1260
   agaccttccc ctcctccaat gagtcccgcc aatgccccaa tgcccgttgc cagttcgcct 1320
   tctacggtgg tgagtcgggc taccaccggg ccctgctggg cctgcagatc ttcaatgcct 1380
   tcatgttctt ctggttggcc aacttcgtgc tggcgctggg ccaggtcacg ctggccgggg 1440
   cctttgcctc ctattactgg gccctgcgca agccggacga cctgccggcc ttcccgctct 1500
   tctctgcctt tggccgggcg ctcaggtacc acacaggctc cctggccttt ggngcgctca 1560
   tcctggccat tgtgcagatc atccgtgtga tactcgagta cctggatcag cggctgaaag 1620
   gtgcagagaa caagtttgcc aagtgcctca tgacctgtct caaatgctgc ttctggtgcc 1680
   tggagaagtt catcaaattc cttaatagga atgcctacat catgattgcc atctacggca 1740
   ccaatttctg cacctcggcc aggaatgcct tcttcctgct catgagaaac atcatcagag 1800
   tggctgtcct ggataaagtt actgacttcc tcttcctgtt gggcaaactt ctgatcgttg 1860
   gtagtgtggg gatcctggct ttcttcttct tcacccaccg tatcaggatc gtgcaggata 1920
   cagcaccacc cctcaattat tactgggttc ctatactgac ggtgatcgtt ggctcctact 1980
   tgattgcaca cggtttcttc agcgtctatg gcatgtgtgt ggacacgctg ttcctctgct 2040
   tcttggagga cctggagagg aatgacggct cggccgagag gccttacttc atgtcttcca 2100
   ccctcaagaa actcttgaac aagaccaaca agaaggcagc ggagtcctga aggccccgtg 2160
   ctccccacct ctcaaggagt ctcatgccgc agggtgctca gtagctgggt ctgttccccc 2220
   agccccttgg gttcacctga agtcctatca ctgccgctct gcccctcccc atgagccaga 2280
   tcccaccagt ttctggacgt ggagagtctg gggcatctcc ttcttatgcc aaggggcgct 2340
   tggagttttc atggctgccc ctccagactg cgagaaacaa gtaaaaaccc wttggggcct 2400
   cttgatgtct gggatggcac gtggcccgac ctccacaagc tccctcatgc ttcctgtccc 2460
   ccgcttacac gacaacgggc cagaccacag gaaggacggt gtttgtgtct gagggagctg 2520
   ctggccacag tgaacaccca cgtttattcc tgcctgctcc ggccaggact gaaccccttc 2580
   tccacacctg aacagttggc tcaagggcca ccagaagcat ttctttatta ttattatttt 2640
   ttaacctgga catgcattaa agggtctatt agctttcttt yncgtctgtc tcaacagctg 2700
   anatnggggc cgccaaggag tgcctttcct tttgcttcct tcntaggttg gagttaacgg 2760
   gtgggaagtt ttttttccca ngtgggggtg ttttcctggt tgggaagg 2808
<210> 3
   <211> 654
   <212> PRT
   <213> Homo sapiens
<220>
   <223> hCTL1b
<400> 3
   Met Gly Cys Cys Ser Ser Ala Ser Ser Ala Ala Gln Ser Ser Lys Arg
   1               5                   10                  15
   Glu Trp Lys Pro Leu Glu Asp Arg Ser Cys Thr Asp Ile Pro Trp Leu
               20                  25                  30
   Leu Leu Phe Ile Leu Phe Cys Ile Gly Met Gly Phe Ile Cys Gly Phe
           35                  40                  45
   Ser Ile Ala Thr Gly Ala Ala Ala Arg Leu Val Ser Gly Tyr Asp Ser
       50                  55                  60
   Tyr Gly Asn Ile Arg Gly Gln Lys Asn Thr Lys Leu Glu Ala Ile Pro
   65                  70                  75                  80
   Asn Ser Gly Met Asp His Thr Gln Arg Lys Tyr Val Phe Phe Leu Asp
                   85                  90                  95
   Pro Cys Asn Leu Asp Leu Ile Asn Arg Lys Ile Lys Ser Val Ala Leu
               100                 105                 110
   Cys Val Ala Ala Cys Pro Arg Gln Glu Leu Lys Thr Leu Ser Asp Val
           115                 120                 125
   Gln Lys Phe Ala Glu Ile Asn Gly Ser Ala Leu Cys Ser Tyr Asn Leu
       130                 135                 140
   Lys Pro Ser Glu Tyr Thr Thr Ser Pro Lys Ser Ser Val Leu Cys Pro
   145                 150                 155                 160
   Lys Leu Pro Val Pro Ala Ser Ala Pro Ile Pro Phe Phe His Arg Cys
                   165                 170                 175
   Ala Pro Val Asn Ile Ser Cys Tyr Ala Lys Phe Ala Glu Ala Leu Ile
               180                 185                 190
   Thr Phe Val Ser Asp Asn Ser Val Leu His Arg Leu Ile Ser Gly Val
           195                 200                 205
   Met Thr Ser Lys Glu Ile Ile Leu Gly Leu Cys Leu Leu Ser Leu Val
       210                 215                 220
   Leu Ser Met Ile Leu Met Val Ile Ile Arg Tyr Ile Ser Arg Val Leu
   225                 230                 235                 240
   Val Trp Ile Leu Thr Ile Leu Val Ile Leu Gly Ser Leu Gly Gly Thr
                   245                 250                 255
   Gly Val Leu Trp Trp Leu Tyr Ala Lys Gln Arg Arg Ser Pro Lys Glu
               260                 265                 270
   Thr Val Thr Pro Glu Gln Leu Gln Ile Ala Glu Asp Asn Leu Arg Ala
           275                 280                 285
   Leu Leu Ile Tyr Ala Ile Ser Ala Thr Val Phe Thr Val Ile Leu Phe
       290                 295                 300
   Leu Ile Met Leu Val Met Arg Lys Arg Val Ala Leu Thr Ile Ala Leu
   305                 310                 315                 320
   Phe His Val Ala Gly Lys Val Phe Ile His Leu Pro Leu Leu Val Phe
                   325                 330                 335
   Gln Pro Phe Trp Thr Phe Phe Ala Leu Val Leu Phe Trp Val Tyr Trp
               340                 345                 350
   Ile Met Thr Leu Leu Phe Leu Gly Thr Thr Gly Ser Pro Val Gln Asn
           355                 360                 365
   Glu Gln Gly Phe Val Glu Phe Lys Ile Ser Gly Pro Leu Gln Tyr Met
       370                 375                 380
   Trp Trp Tyr His Val Val Gly Leu Ile Trp Ile Ser Glu Phe Ile Leu
   385                 390                 395                 400
   Ala Cys Gln Gln Met Thr Val Ala Gly Ala Val Val Thr Tyr Tyr Phe
                   405                 410                 415
   Thr Arg Asp Lys Arg Asn Leu Pro Phe Thr Pro Ile Leu Ala Ser Val
               420                 425                 430
   Asn Arg Leu Ile Arg Tyr His Leu Gly Thr Val Ala Lys Gly Ser Phe
           435                 440                 445
   Ile Ile Thr Leu Val Lys Ile Pro Arg Met Ile Leu Met Tyr Ile His
       450                 455                 460
   Ser Gln Leu Lys Gly Lys Glu Asn Ala Cys Ala Arg Cys Val Leu Lys
   465                 470                 475                 480
   Ser Cys Ile Cys Cys Leu Trp Cys Leu Glu Lys Cys Leu Asn Tyr Leu
                   485                 490                 495
   Asn Gln Asn Ala Tyr Thr Ala Thr Ala Ile Asn Ser Thr Asn Phe Cys
               500                 505                 510
   Thr Ser Ala Lys Asp Ala Phe Val Ile Leu Val Glu Asn Ala Leu Arg
           515                 520                 525
   Val Ala Thr Ile Asn Thr Val Gly Asp Phe Met Leu Phe Leu Gly Lys
       530                 535                 540
   Val Leu Ile Val Cys Ser Thr Gly Leu Ala Gly Ile Met Leu Leu Asn
   545                 550                 555                 560
   Tyr Gln Gln Asp Tyr Thr Val Trp Val Leu Pro Leu Ile Ile Val Cys
                   565                 570                 575
   Leu Phe Ala Phe Leu Val Ala His Cys Phe Leu Ser Ile Tyr Glu Met
               580                 585                 590
   Val Val Asp Val Leu Phe Leu Cys Phe Ala Ile Asp Thr Lys Tyr Asn
           595                 600                 605
   Asp Gly Ser Pro Gly Arg Glu Phe Tyr Met Asp Lys Val Leu Met Glu
       610                 615                 620
   Phe Val Glu Asn Ser Arg Lys Ala Met Lys Glu Ala Gly Lys Gly Gly
   625                 630                 635                 640
   Val Ala Asp Ser Arg Glu Leu Lys Pro Met Leu Lys Lys Arg
                   645                 650
<210> 4
   <211> 706
   <212> PRT
   <213> Homo sapiens
<220>
   <223> hCTL2
<400> 4
   Met Gly Asp Glu Arg Pro His Tyr Tyr Gly Lys His Gly Thr Pro Gln
    1               5                  10                  15
   Lys Tyr Asp Pro Thr Phe Lys Gly Pro Ile Tyr Asn Arg Gly Cys Thr
               20                  25                  30
   Asp Ile Ile Cys Cys Val Phe Leu Leu Leu Ala Ile Val Gly Tyr Val
           35                  40                  45
   Ala Val Gly Ile Ile Ala Trp Thr His Gly Asp Pro Arg Lys Val Ile
       50                  55                  60
   Tyr Pro Thr Asp Ser Arg Gly Glu Phe Cys Gly Gln Lys Gly Thr Lys
   65                  70                  75                      80
   Asn Glu Asn Lys Pro Tyr Leu Phe Tyr Phe Asn Ile Val Lys Cys Ala
                   85                  90                  95
   Ser Pro Leu Val Leu Leu Glu Phe Gln Cys Pro Thr Pro Gln Ile Cys
               100                 105                 110
   Val Glu Lys Cys Pro Asp Arg Tyr Leu Thr Tyr Leu Asn Ala Arg Ser
           115                 120                 125
   Ser Arg Asp Phe Glu Tyr Tyr Lys Gln Phe Cys Val Pro Gly Phe Lys
       130                 135                 140
   Asn Asn Lys Gly Val Ala Glu Val Leu Arg Asp Gly Asp Cys Pro Ala
   145                 150                 155                 160
   Val Leu Ile Pro Ser Lys Pro Leu Ala Arg Arg Cys Phe Pro Ala Ile
                   165                 170                 175
   His Ala Tyr Lys Gly Val Leu Met Val Gly Asn Glu Thr Thr Tyr Glu
               180                 185                 190
   Asp Gly His Gly Ser Arg Lys Asn Ile Thr Asp Leu Val Glu Gly Ala
           195                 200                 205
   Lys Lys Ala Asn Gly Val Leu Glu Ala Arg Gln Leu Ala Met Arg Ile
       210                 215                 220
   Phe Glu Asp Tyr Thr Val Ser Trp Tyr Trp Ile Ile Ile Gly Leu Val
   225                 230                 235                 240
   Ile Ala Met Ala Met Ser Leu Leu Phe Ile Ile Leu Leu Arg Phe Leu
                   245                 250                 255
   Ala Gly Ile Met Val Trp Val Met Ile Ile Met Val Ile Leu Val Leu
               260                 265                 270
   Gly Tyr Gly Ile Phe His Cys Tyr Met Glu Tyr Ser Arg Leu Arg Gly
           275                 280                 285
   Glu Ala Gly Ser Asp Val Ser Leu Val Asp Leu Gly Phe Gln Thr Asp
       290                 295                 300
   Phe Arg Val Tyr Leu His Leu Arg Gln Thr Trp Leu Ala Phe Met Ile
   305                 310                 315                 320
   Ile Leu Ser Ile Leu Glu Val Ile Ile Ile Leu Leu Leu Ile Phe Leu
                   325                 330                 335
   Arg Lys Arg Ile Leu Ile Ala Ile Ala Leu Ile Lys Glu Ala Ser Arg
               340                 345                 350
   Ala Val Gly Tyr Val Met Cys Ser Leu Leu Tyr Pro Leu Val Thr Phe
           355                 360                 365
   Phe Leu Leu Cys Leu Cys Ile Ala Tyr Trp Ala Ser Thr Ala Val Phe
       370                 375                 380
   Leu Ser Thr Ser Asn Glu Ala Val Tyr Lys Ile Phe Asp Asp Ser Pro
   385                 390                 395                 400
   Cys Pro Phe Thr Ala Lys Thr Cys Asn Pro Glu Thr Phe Pro Ser Ser
                   405                 410                 415
   Asn Glu Ser Arg Gln Cys Pro Asn Ala Arg Cys Gln Phe Ala Phe Tyr
               420                 425                 430
   Gly Gly Glu Ser Gly Tyr His Arg Ala Leu Leu Gly Leu Gln Ile Phe
           435                 440                 445
   Asn Ala Phe Met Phe Phe Trp Leu Ala Asn Phe Val Leu Ala Leu Gly
   450 455 460
   Gln Val Thr Leu Ala Gly Ala Phe Ala Ser Tyr Tyr Trp Ala Leu Arg
   465 470 475 480
   Lys Pro Asp Asp Leu Pro Ala Phe Pro Leu Phe Ser Ala Phe Gly Arg
   485 490 495
   Ala Leu Arg Tyr His Thr Gly Ser Leu Ala Phe Val Ala Leu Ile Leu
   500 505 510
   Ala Ile Val Gln Ile Ile Arg Val Ile Leu Glu Tyr Leu Asp Gln Arg
   515 520 525
   Leu Lys Gly Ala Glu Asn Lys Phe Ala Lys Cys Leu Met Thr Cys Leu
   530 535 540
   Lys Cys Cys Phe Trp Cys Leu Glu Lys Phe Ile Lys Phe Leu Asn Arg
   545 550 555 560
   Asn Ala Tyr Ile Met Ile Ala Ile Tyr Gly Thr Asn Phe Cys Thr Ser
   565 570 575
   Ala Arg Asn Ala Phe Phe Leu Leu Met Arg Asn Ile Ile Arg Val Ala
   580 585 590
   Val Leu Asp Lys Val Thr Asp Phe Leu Phe Leu Leu Gly Lys Leu Leu
   595 600 605
   Ile Val Gly Ser Val Gly Ile Leu Ala Phe Phe Phe Phe Thr His Arg
   610 615 620
   Ile Arg Ile Val Gln Asp Thr Ala Pro Pro Leu Asn Tyr Tyr Trp Val
   625 630 635 640
   Pro Ile Leu Thr Val Ile Val Gly Ser Tyr Leu Ile Ala His Gly Phe
   645 650 655
   Phe Ser Val Tyr Gly Met Cys Val Asp Thr Leu Phe Leu Cys Phe Leu
   660 665 670
   Glu Asp Leu Glu Arg Asn Asp Gly Ser Ala Glu Arg Pro Tyr Phe Met
   675 680 685
   Ser Ser Thr Leu Lys Lys Leu Leu Asn Lys Thr Asn Lys Lys Ala Ala
   690 695 700
   Glu Ser
   705
<210> 5
   <211> 24
   <212> ADN
   <213> Séquence artificielle
<220>
   <223> Amorces PCR pour l'actine de levure directes et réverses.
<400> 5
   caaaattggc tagagaaaca accg 24
<210> 6
   <211> 23
   <212> ADN
   <213> Séquence artificielle
<220>
   <223> Amorces PCR pour l'actine de levures réverses.
<400> 6
   aaagaacaat ggccttatac agg 23
<210> 7
   <211> 24
   <212> ADN
   <213> Séquence artificielle
<220>
   <223> Amorces directes utilisées pour localiser la région de codage du gène d'hTCL1.
<400> 7
   catgtggtgg taccatgtgg tggg 24
<210> 8
   <211> 24
   <212> ADN
   <213> Séquence artificielle
<220>
   <223> Amorces réverses utilisées pour localiser la région de codage du gène d'hTCL1.
<400> 8
   cgaataaggc gatttactga tgcc 24
<210> 9
   <211> 657
   <212> PRT
   <213> Homo sapiens
<220>
   <223> hCTL1a
<400> 9
   Met Gly Cys Cys Ser Ser Ala Ser Ser Ala Ala Gln Ser Ser Lys Arg
   1               5                   10                  15
   Glu Trp Lys Pro Leu Glu Asp Arg Ser Cys Thr Asp Ile Pro Trp Leu
               20                  25                  30
   Leu Leu Phe Ile Leu Phe Cys Ile Gly Met Gly Phe Ile Cys Gly Phe
   35 40 45
   Ser Ile Ala Thr Gly Ala Ala Ala Arg Leu Val Ser Gly Tyr Asp Ser
   50 55 60
   Tyr Gly Asn Ile Arg Gly Gln Lys Asn Thr Lys Leu Glu Ala Ile Pro
   65 70 75 80
   Asn Ser Gly Met Asp His Thr Gln Arg Lys Tyr Val Phe Phe Leu Asp
   85 90 95
   Pro Cys Asn Leu Asp Leu Ile Asn Arg Lys Ile Lys Ser Val Ala Leu
   100 105 110
   Cys Val Ala Ala Cys Pro Arg Gln Glu Leu Lys Thr Leu Ser Asp Val
   115 120 125
   Gln Lys Phe Ala Glu Ile Asn Gly Ser Ala Leu Cys Ser Tyr Asn Leu
   130 135 140
   Lys Pro Ser Glu Tyr Thr Thr Ser Pro Lys Ser Ser Val Leu Cys Pro
   145 150 155 160
   Lys Leu Pro Val Pro Ala Ser Ala Pro Ile Pro Phe Phe His Arg Cys
   165 170 175
   Ala Pro Val Asn Ile Ser Cys Tyr Ala Lys Phe Ala Glu Ala Leu Ile
   180 185 190
   Thr Phe Val Ser Asp Asn Ser Val Leu His Arg Leu Ile Ser Gly Val
   195 200 205
   Met Thr Ser Lys Glu Ile Ile Leu Gly Leu Cys Leu Leu Ser Leu Val
   210 215 220
   Leu Ser Met Ile Leu Met Val Ile Ile Arg Tyr Ile Ser Arg Val Leu
   225 230 235 240
   Val Trp Ile Leu Thr Ile Leu Val Ile Leu Gly Ser Leu Gly Gly Thr
   245 250 255
   Gly Val Leu Trp Trp Leu Tyr Ala Lys Gln Arg Arg Ser Pro Lys Glu
   260 265 270
   Thr Val Thr Pro Glu Gln Leu Gln Ile Ala Glu Asp Asn Leu Arg Ala
   275 280 285
   Leu Leu Ile Tyr Ala Ile Ser Ala Thr Val Phe Thr Val Ile Leu Phe
   290 295 300
   Leu Ile Met Leu Val Met Arg Lys Arg Val Ala Leu Thr Ile Ala Leu
   305 310 315 320
   Phe His Val Ala Gly Lys Val Phe Ile His Leu Pro Leu Leu Val Phe
   325 330 335
   Gln Pro Phe Trp Thr Phe Phe Ala Leu Val Leu Phe Trp Val Tyr Trp
   340 345 350
   Ile Met Thr Leu Leu Phe Leu Gly Thr Thr Gly Ser Pro Val Gln Asn
           355                 360                 365
   Glu Gln Gly Phe Val Glu Phe Lys Ile Ser Gly Pro Leu Gln Tyr Met
       370                 375                 380
   Trp Trp Tyr His Val Val Gly Leu Ile Trp Ile Ser Glu Phe Ile Leu
   385                 390                 395                 400
   Ala Cys Gln Gln Met Thr Val Ala Gly Ala Val Val Thr Tyr Tyr Phe
                   405                 410                 415
   Thr Arg Asp Lys Arg Asn Leu Pro Phe Thr Pro Ile Leu Ala Ser Val
               420                 425                 430
   Asn Arg Leu Ile Arg Tyr His Leu Gly Thr Val Ala Lys Gly Ser Phe
           435                 440                 445
   Ile Ile Thr Leu Val Lys Ile Pro Arg Met Ile Leu Met Tyr Ile His
       450                 455                 460
   Ser Gln Leu Lys Gly Lys Glu Asn Ala Cys Ala Arg Cys Val Leu Lys
   465                 470                 475                 480
   Ser Cys Ile Cys Cys Leu Trp Cys Leu Glu Lys Cys Leu Asn Tyr Leu
                   485                 490                 495
   Asn Gln Asn Ala Tyr Thr Ala Thr Ala Ile Asn Ser Thr Asn Phe Cys
               500                 505                 510
   Thr Ser Ala Lys Asp Ala Phe Val Ile Leu Val Glu Asn Ala Leu Arg
           515                 520                 525
   Val Ala Thr Ile Asn Thr Val Gly Asp Phe Met Leu Phe Leu Gly Lys
       530                 535                 540
   Val Leu Ile Val Cys Ser Thr Gly Leu Ala Gly Ile Met Leu Leu Asn
   545                 550                 555                 560
   Tyr Gln Gln Asp Tyr Thr Val Trp Val Leu Pro Leu Ile Ile Val Cys
                   565                 570                 575
   Leu Phe Ala Phe Leu Val Ala His Cys Phe Leu Ser Ile Tyr Glu Met
               580                 585                 590
   Val Val Asp Val Leu Phe Leu Cys Phe Ala Ile Asp Thr Lys Tyr Asn
           595                 600                 605
   Asp Gly Ser Pro Gly Arg Glu Phe Tyr Met Asp Lys Val Leu Met Glu
       610                 615                 620
   Phe Val Glu Asn Ser Arg Lys Ala Met Lys Glu Ala Gly Lys Gly Gly
   625                 630                 635                 640
   Val Ala Asp Ser Arg Glu Leu Lys Pro Met Ala Ser Gly Ala Ser Ser
                   645                 650                 655
   Ala